# EUROPEAN PATENT SPECIFICATION

(11) **EP 3 646 900 B1**
(45) Date of publication and mention of the grant of the patent: **21.05.2025**
(21) Application number: 18825075.7
(22) Date of filing: 29.06.2018
(51) Int. Cl.: A61L 31/06, A61B 1/005, A61L 31/12, C08J 7/04, C08K 5/3435, C08L 77/12, F16L 11/08, G02B 23/24, A61L 29/08, B32B 27/36, B29C 48/09, B29C 48/21, B29C 48/151, B29C 48/92, B29C 48/34, B32B 15/02, B32B 15/08, C08J 7/043, C08K 5/52, C09D 167/02, F16L 55/11

(54) **ENDOSCOPE FLEXIBLE TUBE, ENDOSCOPE-TYPE MEDICAL DEVICE, RESIN COMPOSITION FOR COATING ENDOSCOPE FLEXIBLE TUBE SUBSTRATE, AND RESIN COMPOSITION SET FOR COATING ENDOSCOPE FLEXIBLE TUBE SUBSTRATE**
ENDOSKOPSCHLAUCH, ENDOSKOPARTIGES MEDIZINPRODUKT, HARZZUSAMMENSETZUNG ZUR BESCHICHTUNG EINES ENDOSKOPSCHLAUCHSUBSTRATS UND HARZZUSAMMENSETZUNGSSET ZUR BESCHICHTUNG EINES ENDOSKOPSCHLAUCHSUBSTRATS
TUBE SOUPLE D'ENDOSCOPE, DISPOSITIF MÉDICAL DE TYPE ENDOSCOPE, COMPOSITION DE RÉSINE POUR LE REVÊTEMENT D'UN SUBSTRAT DE TUBE SOUPLE D'ENDOSCOPE, ET COMPOSITION DE RÉSINE DURCIE POUR LE REVÊTEMENT D'UN SUBSTRAT DE TUBE SOUPLE D'ENDOSCOPE

(30) Priority: 30.06.2017 JP 2017129905
(43) Date of publication of application: 06.05.2020
(73) Proprietor: FUJIFILM Corporation, Tokyo 106-8620 (JP)
(72) Inventor: FURUKAWA, Kazushi, Ashigarakami-gun Kanagawa 258-8577 (JP); NAKAI, Yoshihiro, Ashigarakami-gun Kanagawa 258-8577 (JP)
(74) Representative: Hoffmann Eitle
(86) International application number: PCT/JP2018/024966
(87) International publication number: WO 2019/004473

(56) References cited:
- EP-A1- 2 980 466
- WO-A1-2012/053341
- JP-A- 2008 507 632
- JP-A- 2011 072 391
- JP-A- 2011 527 357
- JP-A- 2014 188 217
- JP-A- 2015 016 261
- JP-A- 2015 154 924
- JP-A- H02 283 346
- JP-A- H11 158 362

## Description

### BACKGROUND OF THE INVENTION

### 1. Field of the Invention

The present invention is defined by the claims and relates to flexible tubes for endoscopes, endoscopic medical devices, and sets of resin compositions for covering flexible tube substrates for endoscopes.

### 2. Description of the Related Art

Endoscopes are medical devices for examination of a patient's body cavity. Since an endoscope is inserted and used in a body cavity, an endoscope that does not damage an organ or cause pain or discomfort to a patient is desirable. To meet this need, a spiral tube formed by spirally winding a flexible metal strip is used as a flexible tube that forms the insertion section of an endoscope. In addition, the periphery of the spiral tube is covered with a soft resin and is further covered with a topcoat layer so as not to cause stimulation or damage to the surface of a body cavity such as the esophagus or intestine.

Endoscopes are repeatedly used and therefore require cleaning and chemical disinfection after each use. Accordingly, technological development has been made to improve the chemical resistance of endoscopes. For example, JP2014-188217A describes a flexible tube, for an endoscope, having a resin layer composed of at least two layers, including a first layer including at least one elastomer or chain-extended derivative thereof selected from the group consisting of polyester elastomers, polyurethane elastomers, and polyamide elastomers and a second layer including chain-extended derivatives of at least two elastomers selected from the group consisting of polyester elastomers, polyurethane elastomers, and polyamide elastomers. This flexible tube is reported to have high resistance to washing solutions, to exhibit less change in physical properties with temperature (temperature dependence), and to have good adhesiveness between the resin layer and the topcoat layer.

JP2015-16261A describes a flexible tube, for an endoscope, having a resin layer including a layer containing a polyester elastomer and a hindered phenol compound or hindered amine compound. This flexible tube is reported to have the desired properties for endoscopes, including good flexibility, elasticity, and bending durability, as well as good resistance to various disinfectants.

EP 2 980 466 A1 describes a flexible tube having a cylindrical flexible tube base that has flexibility and a resin layer that coats the flexible tube base, in which the resin layer is configured by at least two layers of a first layer that contains a specific resin and a second layer that contains a specific resin, or in which the resin layer is a single layer or multiple layers of two or more layers, and a layer A that is any of the resin layers includes polyester elastomers, and, hindered phenol compounds or hindered amine compounds.

JP H11 158362 A describes a tubular article that is obtained by molding a polyester elastomer resin composition having transmittance or transparency and a haze value (degree of cloudiness) of <85% measured with a 2 mm thick sheet. The polyester elastomer resin composition comprises (A) 100 parts by weight of a polyester block copolymer consisting mainly of 10-50 wt.% of a high melting point crystalline segment comprising crystalline aromatic polyester units and 90-50 wt.% of a low melting point polymer segment comprising aliphatic polyether units and/or aliphatic polyester units and (B) 0.2-20 parts by weight of an ethylenic copolymer having alkali metal carboxylate groups on side chains (preferably a copolymer obtained by neutralizing an ethylene-unsaturated carboxylic acid copolymer with sodium).

### SUMMARY OF THE INVENTION

The properties of flexible tubes for endoscopes have been improved by techniques such as those described in the above patent documents. On the other hand, the requirements on the handleability, durability, and other properties of flexible tubes for endoscopes are becoming more stringent year by year. In view of the foregoing, an object of the present invention is to provide a flexible tube, for an endoscope, that contains sufficiently few defects in the resin layer after molding, that has the desired sufficient chemical resistance, and that can achieve a higher adhesiveness between the topcoat layer and the resin layer, an endoscopic medical device including such a flexible tube for an endoscope, and a set of resin compositions that are suitable for forming a resin layer of such a flexible tube for an endoscope.

The foregoing object is achieved by the following solutions:
(1) A flexible tube for an endoscope has a flexible tube substrate, for an endoscope, that is flexible and tubular and a resin layer covering the flexible tube substrate for an endoscope,
   wherein the resin layer includes one or more layers, the layers including a layer A including a polyester elastomer (a) as a resin component, at least one of a phosphorus-containing compound (b1) or a thioether compound (b2), and a hindered amine compound (c) ,
   the phosphorus-containing compound (b1) is a compound having a structure represented by general formula (1),
   the thioether compound (b2) is a compound having a structure represented by general formula (2), and
   the hindered amine compound (c) is a compound having a structural moiety represented by general formula (3): 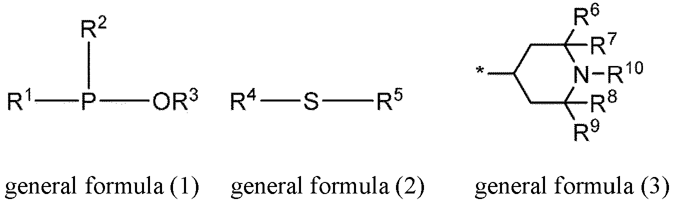 wherein
      in general formula (1), R¹ and R² represent a substituted or unsubstituted alkyl group, a substituted or unsubstituted aryl group, a substituted or unsubstituted alkoxy group, a substituted or unsubstituted aryloxy group, or a halogen atom; R³ represents a substituted or unsubstituted alkyl group or a substituted or unsubstituted aryl group; and at least two of R¹, R², or R³ may be linked to each other via a divalent or higher-valent group or a single bond,
      in general formula (2), R⁴ and R⁵ represent a substituted or unsubstituted alkyl group and may be linked to each other via a divalent or higher-valent group or a single bond, and
      in general formula (3), R⁶ to R⁹ represent a hydrogen atom or an alkyl group having 1 to 12 carbon atoms; R¹⁰ represents a hydrogen atom, an alkyl group having 1 to 18 carbon atoms, or -OR¹¹, wherein R¹¹ represents a hydrogen atom or an alkyl group having 1 to 20 carbon atoms; and * represents a point of attachment;
      the recited alkyl and alkoxy groups are linear, branched or cyclic groups.
(2) A set of resin compositions for covering a flexible tube substrate for an endoscope includes a resin composition (A) including a polyester elastomer (a), at least one of a phosphorus-containing compound (b1) or a thioether compound (b2), and a hindered amine compound (c); and a resin composition (B) including at least one of a polyester elastomer (a1), a polyurethane elastomer (d), or a polyamide elastomer (e),
   wherein the resin composition (A) is for forming an outer layer covering the flexible tube substrate for an endoscope and the resin composition (B) is for forming an inner layer covering the flexible tube substrate for an endoscope, and
   wherein the phosphorus-containing compound (b1) is a compound having a structure represented by general formula (1), the thioether compound (b2) is a compound having a structure represented by general formula (2), and the hindered amine compound (c) is a compound having a structural moiety represented by general formula (3): 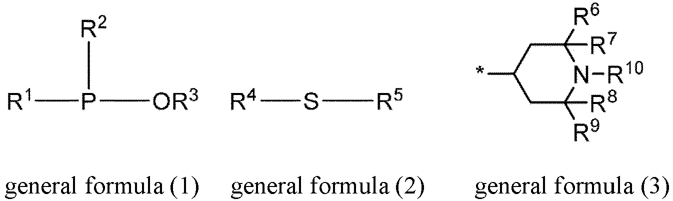 wherein
   in general formula (1), R¹ and R² represent an alkyl group, an aryl group, an alkoxy group, an aryloxy group, or a halogen atom; R³ represents an alkyl group or an aryl group; and at least two of R¹, R², or R³ may be linked to each other via a divalent or higher-valent group or a single bond,
   in general formula (2), R⁴ and R⁵ represent an alkyl group and may be linked to each other via a divalent or higher-valent group or a single bond, and
   in general formula (3), R⁶ to R⁹ represent a hydrogen atom or an alkyl group having 1 to 12 carbon atoms; R¹⁰ represents a hydrogen atom, an alkyl group having 1 to 18 carbon atoms, or -OR¹¹, wherein R¹¹ represents a hydrogen atom or an alkyl group having 1 to 20 carbon atoms; and * represents a point of attachment,
   wherein the alkyl group, the aryl group, the alkoxy group and the aryloxy group represented by R¹ and R² in general formula (1) are a substituted or unsubstituted alkyl group, a substituted or unsubstituted aryl group, a substituted or unsubstituted alkoxy group and a substituted or unsubstituted aryloxy group; the alkyl group and the aryl group represented by R³ in general formula (1) are a substituted or unsubstituted alkyl group and a substituted or unsubstituted aryl group; and the alkyl group represented by R⁴ and R⁵ in general formula (2) is a substituted or unsubstituted alkyl group;
   the recited alkyl and alkoxy groups are linear, branched or cyclic groups.

In the description of the present invention, a "resin component" present in a resin layer refers to an elastomer. "Resin component" may be hereinafter simply referred to as "resin".

In the description of the present invention, if there are a plurality of substituents, linking groups, or the like (hereinafter referred to as "substituent or the like") represented by a particular symbol, or if a plurality of substituents or the like are specified simultaneously or alternatively, it is meant that the individual substituents or the like may be the same or different. In addition, if a plurality of substituents or the like are adjacent to each other, it is meant that they may be linked or fused to each other to form a ring, even if not specified as such.

In the description of the present invention, if it is not explicitly specified whether a substituent (or linking group) is substituted or unsubstituted, it is meant that the group may have any substituent as long as the advantages of the present invention are not impaired. This also applies if it is not explicitly specified whether a compound is substituted or unsubstituted.

The flexible tube for an endoscope according to the present invention contains sufficiently few defects in the resin layer after molding, has the desired sufficient chemical resistance, and can achieve a higher adhesiveness between the topcoat layer and the resin layer. The endoscopic medical device according to the present invention includes the flexible tube for an endoscope with the above superior properties. The set of resin compositions for covering a flexible tube substrate for an endoscope according to the present invention is suitable for use as a material for forming the resin layer of the flexible tube for an endoscope with the above properties.

### BRIEF DESCRIPTION OF THE DRAWINGS

Fig. 1 is an external view illustrating the configuration of an electronic endoscope;
Fig. 2 is a partial sectional view schematically illustrating the configuration of a flexible tube for an endoscope;
Fig. 3 is a block diagram schematically illustrating the configuration of an apparatus for manufacturing the flexible tube for an endoscope; and
Fig. 4 is a sectional view taken along line B-B of Fig. 3.

### DESCRIPTION OF THE PREFERRED EMBODIMENTS

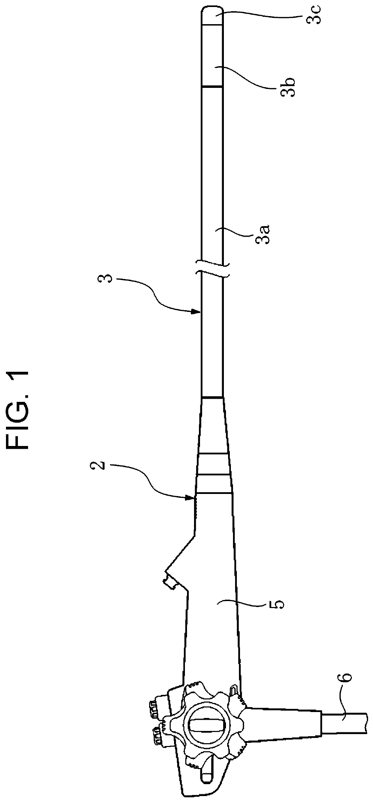

An electronic endoscope will now be described as an example of an endoscopic medical device according to a preferred embodiment of the present invention. Electronic endoscopes incorporate a flexible tube for an endoscope (a flexible tube for an endoscope may be hereinafter simply referred to as "flexible tube") and are widely used as medical devices. In the example shown in Fig. 1, an electronic endoscope 2 includes an insertion section 3 for insertion into a body cavity, a main-body operating section 5 connected to the proximal end portion of the insertion section 3, and a universal cord 6 for connection to a processor device and a light source device. The insertion section 3 is composed of a flexible tube 3a connected to the main-body operating section 5, an angle portion 3b connected to the flexible tube 3a, and a tip portion 3c connected to the distal end of the angle portion 3b and having an imaging device (not shown) built thereinto for imaging a body cavity. The flexible tube 3a, which accounts for most of the length of the insertion section 3, is flexible substantially over the entire length thereof. In particular, the portion to be inserted into an area such as a body cavity has a more flexible structure.

### Flexible Tube

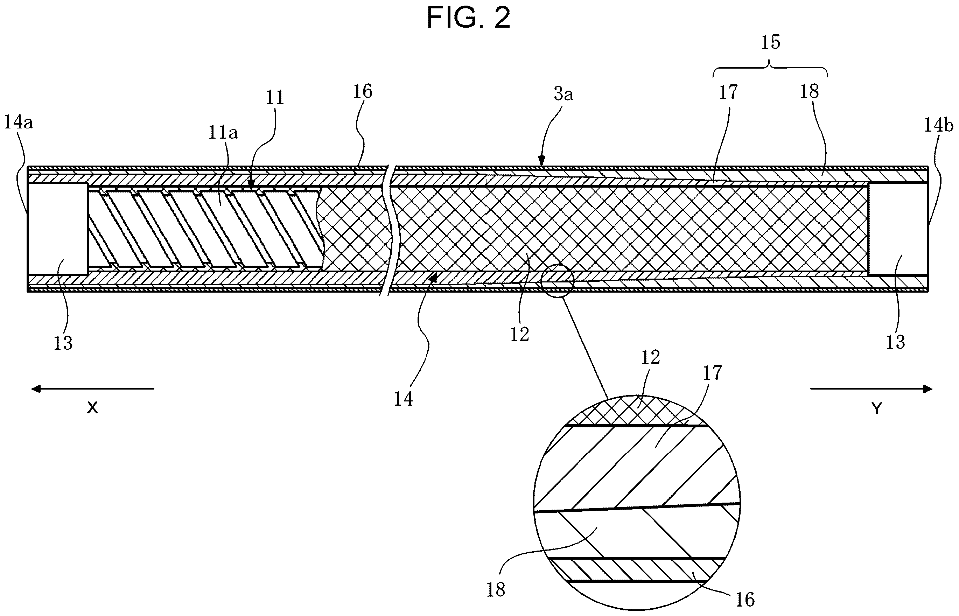

As shown in Fig. 2, the flexible tube 3a (flexible tube for an endoscope) is composed of a flexible tube substrate 14 and a resin layer 15 covering the outer peripheral surface of the flexible tube substrate 14. The flexible tube substrate 14 includes a spiral tube 11 disposed on the innermost side and formed by spirally winding a metal strip 11a, a tubular net 12 covering the spiral tube 11 and formed by weaving metal wires, and caps 13 fitted to both ends. The outer surface of the resin layer 15 is covered with a chemical-resistant coat layer 16 such as one containing fluorine. Although the spiral tube 11 is shown as a single layer, it may be composed of two layers coaxially stacked on top of each other. To clearly illustrate the layer structure, the resin layer 15 and the coat layer 16 are shown as being thick relative to the diameter of the flexible tube substrate 14.

The resin layer 15 according to this embodiment covers the outer peripheral surface of the flexible tube substrate 14. The resin layer 15 has a two-layer configuration including an inner layer 17 covering the entire peripheral surface of the flexible tube substrate 14 about the axis thereof and an outer layer 18 covering the entire peripheral surface of the inner layer 17 about the axis thereof. A soft resin is used as the material for the inner layer 17, whereas a hard resin is used as the material for the outer layer 18. In other preferred embodiments of the present invention, the resin layer 15 may be composed of one layer (a layer A) or three or more layers (including the layer A).

In this embodiment, the resin layer 15 is formed with substantially uniform thickness in the longitudinal direction (axial direction) of the flexible tube substrate 14. The resin layer 15 has a thickness of, for example, 0.2 mm to 1.0 mm. The flexible tube 3a has an outer diameter D of, for example, 11 to 14 mm. The inner layer 17 and the outer layer 18 are formed such that the proportions of the thicknesses of the individual layers 17 and 18 relative to the total thickness of the resin layer 15 vary in the axial direction of the flexible tube substrate 14. Specifically, the proportion of the thickness of the inner layer 17 relative to the total thickness of the resin layer 15 is larger than that of the outer layer 18 on one end 14a side (distal side) of the flexible tube substrate 14 attached to the angle portion 3b. The inner layer 17 gradually becomes thinner from the end 14a toward the other end 14b side (proximal side) attached to the main-body operating section 5. The outer layer 18 is thicker than the inner layer 17 on the other end 14b side.

The proportion of the thickness of the inner layer 17 is maximum at the end 14a in this embodiment. The proportion of the thickness of the outer layer 18 is maximum at the other end 14b in this embodiment. The proportion of the thickness of the inner layer 17 to the thickness of the outer layer 18 is 9:1 at the end 14a and is 1:9 at the other end 14b. The proportion of the thickness of the inner layer 17 to the thickness of the outer layer 18 varies so as to be reversed between both ends 14a and 14b. Thus, there is a difference in hardness between the end 14a side and the other end 14b side of the flexible tube 3a, and the softness varies in the axial direction such that the flexible tube 3a is softer on the end 14a side and is harder on the other end 14b side. Preferably, the proportion of the thickness of the inner layer to the thickness of the outer layer is 5:95 to 40:60 (inner layer:outer layer) at one end and is 95:5 to 60:40 (inner layer:outer layer) at the other end.

As in the example above, it is preferred that the proportion of the thickness of the inner layer 17 to the thickness of the outer layer 18 be 5:95 to 95:5. Within this range, the amount of resin extruded can be more precisely controlled for the thinner layer.

The difference in modulus at 100% elongation, which is a measure of hardness after molding, between the soft resin used for the inner layer 17 and the hard resin used for the outer layer 18 is preferably 1 MPa or more, more preferably 3 MPa or more. The difference in melt viscosity at a molding temperature of 150°C to 300°C, which is a measure of the fluidity of a molten resin, between the soft resin used for the inner layer 17 and the hard resin used for the outer layer 18 is preferably 2,500 Pa·s or less. This ensures that the resin layer 15 composed of the inner layer 17 and the outer layer 18 has both good molding accuracy and the required hardness difference between the distal side and the proximal side.

### Method for Manufacturing Flexible Tube

An example method for manufacturing a flexible tube including a resin layer having a two-layer structure composed of an inner layer and an outer layer will hereinafter be described. A flexible tube including a resin layer composed of one layer or three or more layers can also be manufactured as in the method described below.

To form a resin layer composed of at least two layers including an inner layer and an outer layer, it is preferred to
(i) provide a first resin material for the inner layer;
(ii) provide a second resin material for the outer layer; and
(iii) melt-kneading the first resin material and the second resin material and extrude them onto the periphery of the flexible tube substrate to cover the flexible tube substrate with the resin layer.

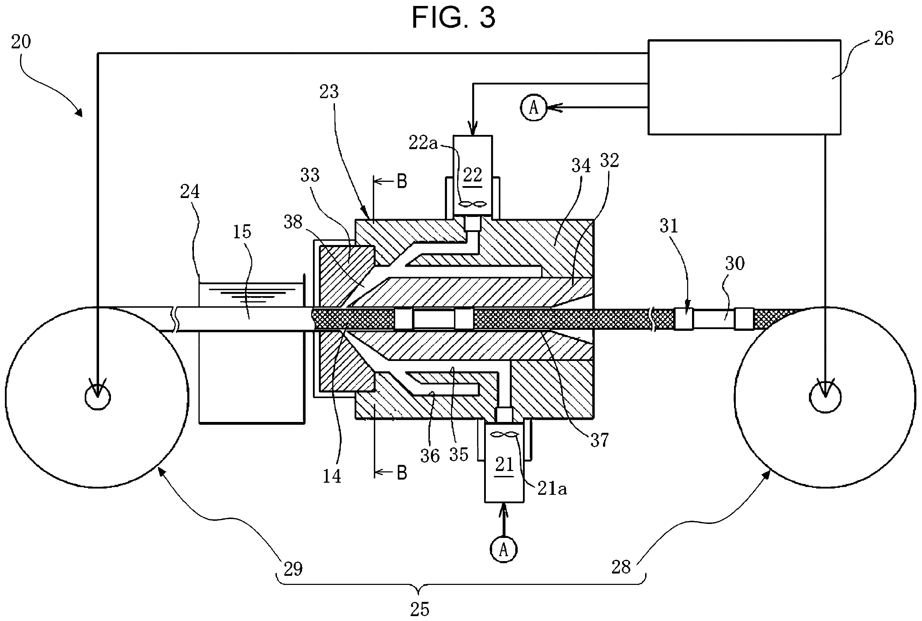

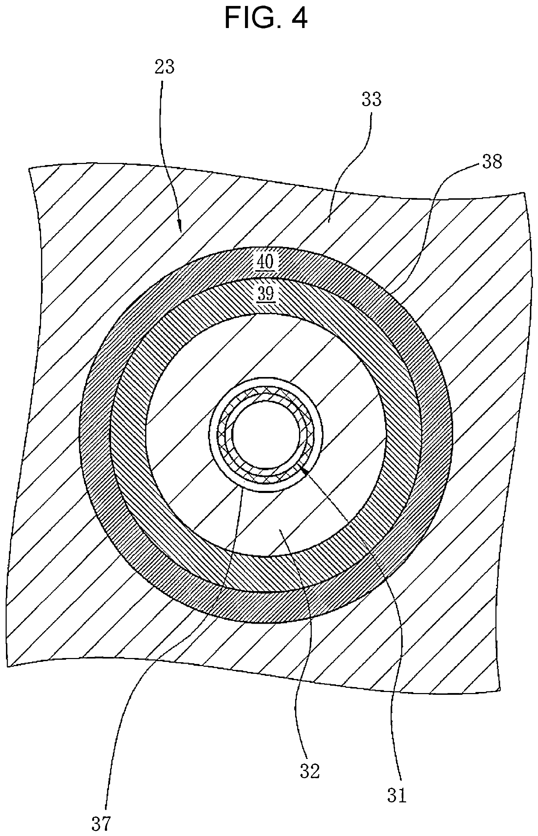

A method for manufacturing the flexible tube 3a (Figs. 1 and 2) will now be described with reference to Figs. 3 and 4. The resin layer 15 is preferably molded using a continuous molding machine. It is preferred to use a continuous molding machine 20 composed of known extrusion units 21 and 22 composed of parts such as hoppers and screws 21a and 22a, a head unit 23 for covering the outer peripheral surface of the flexible tube substrate 14 with the resin layer 15, a cooling unit 24, a transport unit 25 (a feed drum 28 and a take-up drum 29) that transports a continuous flexible tube substrate 31 to the head unit 23, and a control unit 26 that controls these units. The head unit 23 is preferably composed of a nipple 32, a die 33, and a support 34 fixedly supporting them. An example configuration of such a machine that can be used is shown in, for example, Figs. 3 to 5 of JP2011-72391A.

The interior of the die 33 is preferably heated to a predetermined molding temperature. The molding temperature is preferably set within the range of 150°C to 300°C. A soft resin 39 and a hard resin 40 can be heated to a high temperature by the heating temperature control of a heating unit within the machine. Additionally, as the rotational speeds of the screws 21a and 22a become higher, the soft resin 39 and the hard resin 40 can be heated to a higher temperature, thereby increasing their fluidity. During this process, the molding thicknesses of the inner layer 17 and the outer layer 18 can be adjusted by changing the amounts of the molten soft resin 39 and hard resin 40 ejected while transporting the continuous flexible tube substrate 31 at constant speed.

The process of molding the resin layer 15 onto the continuous flexible tube substrate 31 using the continuous molding machine 20 will now be described. When the continuous molding machine 20 performs the molding step, the molten soft resin 39 and hard resin 40 are extruded from the extrusion units 21 and 22 into the head unit 23. At the same time, the transport unit 25 operates to transport the continuous flexible tube substrate 31 to the head unit 23. During this process, the extrusion units 21 and 22 constantly extrude and feed the soft resin 39 and the hard resin 40 to the head unit 23, and the soft resin 39 and the hard resin 40 extruded from the extrusion units 21 and 22 into gates 35 and 36 merge together at an edge and, in a stacked state, are fed through a resin passage 38 to a molding passage 37. Thus, a two-layer molded resin layer 15 composed of a stack of an inner layer 17 made of the soft resin 39 and an outer layer 18 made of the hard resin 40 is formed.

The continuous flexible tube substrate 31 is composed of a plurality of flexible tube substrates 14 joined together. The resin layer 15 is continuously molded onto the plurality of flexible tube substrates 14 being transported through the molding passage 37. When the resin layer 15 is molded from the end 14a side (distal side) to the other end 14b side (proximal side) of one flexible tube substrate, the inner layer 17 is thick immediately after the extrusion units 21 and 22 start resin ejection. The proportion of the thickness of the outer layer 18 gradually increases over the middle portion toward the other end 14b side. It is preferred to control the amounts of the resins ejected in this way to achieve the above gradient in the proportion of the thickness of the resin layer 15.

A joint member 30, which is a connecting portion between two flexible tube substrates 14, is used for switching of the amounts of the resins ejected from the extrusion units 21 and 22 by the control unit 26. Specifically, the control unit 26 preferably switches the amounts of the resins ejected from the extrusion units 21 and 22 for transition from the proportion of the thickness on the other end 14b side (proximal side) of one flexible tube substrate 14 to the proportion of the thickness on the end 14a side (distal side) of the next flexible tube substrate 14. When the resin layer 15 is molded from the end 14a side to the other end 14b side of the next flexible tube substrate 14, it is preferred to similarly control the extrusion units 21 and 22 such that the outer layer gradually becomes thicker from one end side toward the other end side.

After the continuous flexible tube substrate 31 having the resin layer 15 molded to the rearmost end is detached from the continuous molding machine 20, the joint members 30 are detached from the flexible tube substrates 14 to separate the continuous flexible tube substrate 31 into the individual flexible tube substrates 14. The resin layer 15 on the separated flexible tube substrates 14 is then coated with the coat layer 16. Thus, flexible tubes 3a are finished. The finished flexible tubes 3a are transported to an electronic endoscope assembly step.

### Resin Layer

Preferably, the resin layer of the flexible tube according to the present invention is composed of one or more layers, and the outermost layer of the resin layer is a layer A (a layer containing a polyester elastomer (a) (resin component), at least one of a phosphorus-containing compound (b1) or a thioether compound (b2) (a phosphorus-containing compound (b1) and/or a thioether compound (b2)), and a hindered amine compound (c)). Here, the "outermost layer" of a resin layer having a single-layer structure refers to the resin layer itself, whereas the "outermost layer" of a resin layer having a multilayer structure composed of two or more layers refers to the surface layer of the resin layer of the flexible tube. The flexible tube according to the present invention preferably has another layer (e.g., a topcoat layer) outside the resin layer.

Since the flexible tube according to the present invention has the resin layer with the above configuration, the flexible tube contains few defects due to the formation of bubbles in the resin layer after molding, can achieve the desired sufficient chemical resistance, and can achieve high adhesiveness between the topcoat layer and the resin layer. Although the mechanism is not fully understood, one possible factor is that at least one of the phosphorus-containing compound (b1) or the thioether compound (b2) inhibits the degradation reaction (oxidation) of the resin due to heat, for example, during flexible tube molding. Specifically, one possible factor is that at least one of the phosphorus-containing compound (b1) or the thioether compound (b2) quickly decomposes oxidative degradation products formed from the resin component and thus inhibits the decomposition of the resin such as the polyester elastomer (a) into oligomers and other products.

If the resin layer of the flexible tube according to the present invention is composed of a plurality of layers, the flexible tube according to the present invention provides the above advantageous effects even if the layer A is not the outermost layer, but is an inner layer or interlayer. One possible factor for this is that inhibiting the degradation reaction of the resin such as the polyester elastomer (a) in the inner layer or interlayer can inhibit the formation of degradation products or the formation of bubbles, or both, in the inner layer or interlayer and can thus inhibit the migration of degradation products into the outer layer or the deformation of the resin forming the outer layer resin due to bubbles, or both.

### Polyester Elastomer (a)

The polyester elastomer (a) used in the present invention may be a common polyester elastomer that is applicable to the formation of flexible tubes.

Specifically, the polyester elastomer (a) used in the present invention is a copolymer composed of hard segments of a crystalline polyester and soft segments of a polyether or a polyester.

Examples of hard segments include polybutylene terephthalate and polyethylene terephthalate.

Examples of soft segments include polyalkylene glycols such as polytetramethylene glycol and polypropylene glycol, bisphenol A ethylene oxide adducts, bisphenol A propylene oxide adducts, and polyesters such as polycaprolactone.

In the present invention, a "polyester elastomer" may have urethane bonds, amide bonds, or both. In this case, of ester bonds, urethane bonds, and amide bonds, ester bonds are present in the largest number. Preferably, a "polyester elastomer" includes no urethane bond or amide bond in the molecule thereof.

Polyester elastomers (a) may be used alone (not part of the invention) or in combination.

### Phosphorus-Containing Compound (b1)

The phosphorus-containing compound (b1) is a compound having a structure represented by general formula (1):

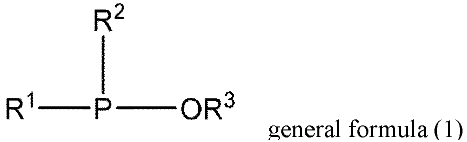

In general formula (1), R¹ and R² represent an alkyl group, an aryl group, an alkoxy group, an aryloxy group, or a halogen atom; R³ represents an alkyl group or an aryl group; and at least two of R¹, R², or R³ may be linked to each other via a divalent or higher-valent group or a single bond.

In the present invention, compounds having a structure represented by general formula (1) include, in addition to compounds represented by general formula (1), compounds (a) and (b) below.
(a) Compounds having a structure in which a monovalent group derived by removing one hydrogen atom from R¹, R², or R³ is linked to at least one of R¹, R², or R³ (R¹, R², and/or R³) of one or more (preferably one to three) other compounds represented by general formula (1) via a divalent or higher-valent group or a single bond; and
(b) compounds having a structure in which a divalent or higher-valent group derived by removing a total of two or more hydrogen atoms from at least one group selected from the group consisting of R¹, R², and R³ (e.g., a divalent group if two hydrogen atoms are removed, or a trivalent group if three hydrogen atoms are removed) is linked to at least one of R¹, R², or R³ of one or more (preferably one to three) other compounds represented by general formula (1) via a divalent or higher-valent group or a single bond.

That is, in the present invention, compounds having a structure represented by general formula (1) are meant to include compounds represented by general formula (1) and compounds having a structure in which a plurality of structures represented by general formula (1) are present in one molecule.

The alkyl groups represented by R¹, R², and R³ in general formula (1) above are linear, branched, or cyclic substituted or unsubstituted alkyl groups. Preferably, the alkyl groups have 1 to 50 carbon atoms, more preferably 1 to 30 carbon atoms, particularly preferably 1 to 20 carbon atoms. Preferred examples include methyl, ethyl, n-propyl, isopropyl, cyclopropyl, butyl, isobutyl, t-butyl, s-butyl, pentyl, isopentyl, neopentyl, t-pentyl, hexyl, cyclohexyl, heptyl, cyclopentyl, octyl, 2-ethylhexyl, nonyl, decyl, dodecyl, tetradecyl, hexadecyl, octadecyl, eicosyl, docosyl, and triacontyl. More preferred are methyl, ethyl, n-propyl, isopropyl, butyl, isobutyl, t-butyl, s-butyl, pentyl, isopentyl, neopentyl, hexyl, cyclohexyl, octyl, 2-ethylhexyl, dodecyl, hexadecyl, and octadecyl. Even more preferred are methyl, ethyl, n-propyl, isopropyl, butyl, t-butyl, pentyl, isopentyl, hexyl, cyclohexyl, octyl, 2-ethylhexyl, dodecyl, hexadecyl, and octadecyl.

The alkyl groups represented by R¹, R², and R³ may further have a substituent. Examples of substituents include halogen atoms, alkyl groups (including cycloalkyl groups), alkenyl groups (including cycloalkenyl groups and bicycloalkenyl groups), alkynyl groups, aryl groups, cyano groups, hydroxy groups, nitro groups, carboxy groups, alkoxy groups, aryloxy groups, acyloxy groups, carbamoyloxy groups, alkoxycarbonyloxy groups, aryloxycarbonyloxy groups, amino groups (including anilino groups), acylamino groups, aminocarbonylamino groups, alkoxycarbonylamino groups, aryloxycarbonylamino groups, acyl groups, aryloxycarbonyl groups, alkoxycarbonyl groups, and carbamoyl groups.

More specifically, examples of substituents include halogen atoms (e.g., chlorine, bromine, and iodine atoms); alkyl groups (which represent linear, branched, or cyclic substituted or unsubstituted alkyl groups, including alkyl groups (preferably alkyl groups having 1 to 30 carbon atoms, e.g., methyl, ethyl, n-propyl, isopropyl, t-butyl, n-octyl, eicosyl, 2-chloroethyl, 2-cyanoethyl, and 2-ethylhexyl), cycloalkyl groups (preferably substituted or unsubstituted cycloalkyl groups having 3 to 30 carbon atoms, e.g., cyclohexyl, cyclopentyl, and 4-n-dodecylcyclohexyl), bicycloalkyl groups (preferably substituted or unsubstituted bicycloalkyl groups having 5 to 30 carbon atoms, i.e., monovalent groups derived by removing one hydrogen atom from bicycloalkanes having 5 to 30 carbon atoms, e.g., bicyclo[1.2.2]heptan-2-yl and bicyclo[2.2.2]octan-3-yl), and structures having larger numbers of rings, including tricyclic structures; this definition of alkyl groups also applies to the alkyl groups of the substituents described below (e.g., the alkyl groups of alkylthio groups)); alkenyl groups (including alkenyl groups (preferably substituted or unsubstituted alkenyl groups having 2 to 30 carbon atoms, e.g., vinyl, allyl, prenyl, geranyl, and oleyl), cycloalkenyl groups (preferably substituted or unsubstituted cycloalkenyl groups having 3 to 30 carbon atoms, i.e., monovalent groups derived by removing one hydrogen atom from cycloalkenes having 3 to 30 carbon atoms, e.g., 2-cyclopenten-1-yl and 2-cyclohexen-1-yl), and bicycloalkenyl groups (substituted or unsubstituted bicycloalkenyl groups, preferably substituted or unsubstituted bicycloalkenyl groups having 5 to 30 carbon atoms, i.e., monovalent groups derived by removing one hydrogen atom from bicycloalkenes having one double bond, e.g., bicyclo[2.2.1]hept-2-en-1-yl and bicyclo[2.2.2]oct-2-en-4-yl));
alkynyl groups (preferably substituted or unsubstituted alkynyl groups having 2 to 30 carbon atoms, e.g., ethynyl, propargyl, and trimethylsilylethynyl); aryl groups (preferably substituted or unsubstituted aryl groups having 6 to 30 carbon atoms, e.g., phenyl, p-tolyl, naphthyl, m-chlorophenyl, and o-hexadecanoylaminophenyl); heterocyclic groups (preferably monovalent groups derived by removing one hydrogen atom from 5- or 6-membered substituted or unsubstituted aromatic or nonaromatic heterocyclic compounds, more preferably 5- or 6-membered aromatic heterocyclic groups having 3 to 30 carbon atoms, e.g., 2-furanyl, 2-thienyl, 2-pyrimidinyl, and 2-benzothiazolinyl); cyano groups; hydroxy groups; nitro groups; carboxy groups; alkoxy groups (preferably substituted or unsubstituted alkoxy groups having 1 to 32 carbon atoms, e.g., methoxy, ethoxy, isopropoxy, t-butoxy, n-octyloxy, and 2-methoxyethoxy; aryloxy groups (preferably substituted or unsubstituted aryloxy groups having 6 to 30 carbon atoms, e.g., phenoxy, 2-methylphenoxy, 4-t-butylphenoxy, 3-nitrophenoxy, and 2-tetradecanoylaminophenoxy); silyloxy groups (preferably silyloxy groups having 3 to 20 carbon atoms, e.g., trimethylsilyloxy and t-butyldimethylsilyloxy); heterocyclic oxy groups (preferably substituted or unsubstituted heterocyclic oxy groups having 2 to 30 carbon atoms, 1-phenyltetrazole-5-oxy and 2-tetrahydropyranyloxy); acyloxy groups (preferably formyloxy groups, substituted or unsubstituted alkylcarbonyloxy groups having 2 to 30 carbon atoms, and substituted or unsubstituted arylcarbonyloxy groups having 6 to 30 carbon atoms, e.g., formyloxy, acetyloxy, pivaloyloxy, stearoyloxy, benzoyloxy, and p-methoxyphenylcarbonyloxy); carbamoyloxy groups (preferably substituted or unsubstituted carbamoyloxy groups having 1 to 30 carbon atoms, e.g., N,N-dimethylcarbamoyloxy, N,N-diethylcarbamoyloxy, morpholinocarbonyloxy, N,N-di-n-octylaminocarbonyloxy, and N-n-octylcarbamoyloxy); alkoxycarbonyloxy groups (preferably substituted or unsubstituted alkoxycarbonyloxy groups having 2 to 30 carbon atoms, e.g., methoxycarbonyloxy, ethoxycarbonyloxy, t-butoxycarbonyloxy, and n-octylcarbonyloxy; aryloxycarbonyloxy groups (preferably substituted or unsubstituted aryloxycarbonyloxy groups having 7 to 30 carbon atoms, e.g., phenoxycarbonyloxy, p-methoxyphenoxycarbonyloxy, and p-n-hexadecyloxyphenoxycarbonyloxy);
amino groups (preferably amino groups, substituted or unsubstituted alkylamino groups having 1 to 30 carbon atoms, and substituted or unsubstituted anilino groups having 6 to 30 carbon atoms, e.g., amino, methylamino, dimethylamino, anilino, N-methyl-anilino, and diphenylamino); acylamino groups (preferably formylamino groups, substituted or unsubstituted alkylcarbonylamino groups having 1 to 30 carbon atoms, and substituted or unsubstituted arylcarbonylamino groups having 6 to 30 carbon atoms, e.g., formylamino, acetylamino, pivaloylamino, lauroylamino, benzoylamino, and 3,4,5-tri-n-octyloxyphenylcarbonylamino); aminocarbonylamino groups (preferably substituted or unsubstituted aminocarbonylamino groups having 1 to 30 carbon atoms, e.g., carbamoylamino, N,N-dimethylaminocarbonylamino, N,N-diethylaminocarbonylamino, and morpholinocarbonylamino); alkoxycarbonylamino groups (preferably substituted or unsubstituted alkoxycarbonylamino groups having 2 to 30 carbon atoms, e.g., methoxycarbonylamino, ethoxycarbonylamino, t-butoxycarbonylamino, n-octadecyloxycarbonylamino, and N-methyl-methoxycarbonylamino); aryloxycarbonylamino groups (preferably substituted or unsubstituted aryloxycarbonylamino groups having 7 to 30 carbon atoms, e.g., phenoxycarbonylamino, p-chlorophenoxycarbonylamino, and m-n-octyloxyphenoxycarbonylamino); sulfamoylamino groups (preferably substituted or unsubstituted sulfamoylamino groups having 0 to 30 carbon atoms, e.g., sulfamoylamino, N,N-dimethylaminosulfonylamino, and N-n-octylaminosulfonylamino); alkylsulfonylamino and arylsulfonylamino groups (preferably substituted or unsubstituted alkylsulfonylamino groups having 1 to 30 carbon atoms and substituted or unsubstituted arylsulfonylamino groups having 6 to 30 carbon atoms, e.g., methylsulfonylamino, butylsulfonylamino, phenylsulfonylamino, 2,3,5-trichlorophenylsulfonylamino, and p-methylphenylsulfonylamino);
sulfanyl groups; alkylthio groups (preferably substituted or unsubstituted alkylthio groups having 1 to 30 carbon atoms, e.g., methylthio, ethylthio, and n-hexadecylthio); arylthio groups (preferably substituted or unsubstituted arylthio groups having 6 to 30 carbon atoms, e.g., phenylthio, p-chlorophenylthio, and m-methoxyphenylthio); heterocyclic thio groups (preferably substituted or unsubstituted heterocyclic thio groups having 2 to 30 carbon atoms, e.g., 2-benzothiazolylthio and 1-phenyltetrazol-5-ylthio); sulfamoyl groups (preferably substituted or unsubstituted sulfamoyl groups having 0 to 30 carbon atoms, e.g., N-ethylsulfamoyl, N-(3-dodecyloxypropyl)sulfamoyl, N,N-dimethylsulfamoyl, N-acetylsulfamoyl, N-benzoylsulfamoyl, and N-(N'-phenylcarbamoyl)sulfamoyl); sulfo groups; alkylsulfinyl and arylsulfinyl groups (preferably substituted or unsubstituted alkylsulfinyl groups having 1 to 30 carbon atoms and substituted or unsubstituted arylsulfinyl groups having 6 to 30 carbon atoms, e.g., methylsulfinyl, ethylsulfinyl, phenylsulfinyl, and p-methylphenylsulfinyl);
alkylsulfonyl and arylsulfonyl groups (preferably substituted or unsubstituted alkylsulfonyl groups having 1 to 30 carbon atoms and substituted or unsubstituted arylsulfonyl groups having 6 to 30 carbon atoms, e.g., methylsulfonyl, ethylsulfonyl, phenylsulfonyl, and p-methylphenylsulfonyl); acyl groups (preferably formyl groups, substituted or unsubstituted alkylcarbonyl groups having 2 to 30 carbon atoms, substituted or unsubstituted arylcarbonyl groups having 7 to 30 carbon atoms, and substituted or unsubstituted heterocyclic carbonyl groups having 4 to 30 carbon atoms in which any of the carbon atoms is attached to the carbonyl group), e.g., acetyl, pivaloyl, 2-chloroacetyl, stearoyl, benzoyl, p-n-octyloxyphenylcarbonyl, 2-pyridylcarbonyl, and 2-furylcarbonyl); aryloxycarbonyl groups (preferably substituted or unsubstituted aryloxycarbonyl groups having 7 to 30 carbon atoms, e.g., phenoxycarbonyl, o-chlorophenoxycarbonyl, m-nitrophenoxycarbonyl, and p-t-butylphenoxycarbonyl); alkoxycarbonyl groups (preferably substituted or unsubstituted alkoxycarbonyl groups having 2 to 30 carbon atoms, e.g., methoxycarbonyl, ethoxycarbonyl, t-butoxycarbonyl, and n-octadecyloxycarbonyl);
carbamoyl groups (preferably substituted or unsubstituted carbamoyl groups having 1 to 30 carbon atoms, e.g., carbamoyl, N-methylcarbamoyl, N,N-dimethylcarbamoyl, N,N-di-n-octylcarbamoyl, and N-(methylsulfonyl)carbamoyl); arylazo and heterocyclic azo groups (preferably substituted or unsubstituted arylazo groups having 6 to 30 carbon atoms and substituted or unsubstituted heterocyclic azo groups having 3 to 30 carbon atoms, e.g., phenylazo, p-chlorophenylazo, and 5-ethylthio-1,3,4-thiadiazol-2-ylazo); imide groups (preferably N-succinimide and N-phthalimide); phosphino groups (preferably substituted or unsubstituted phosphino groups having 2 to 30 carbon atoms, e.g., dimethylphosphino, diphenylphosphino, and methylphenoxyphosphino); phosphinyl groups (preferably substituted or unsubstituted phosphinyl groups having 2 to 30 carbon atoms, e.g., phosphinyl, dioctyloxyphosphinyl, and diethoxyphosphinyl); phosphinyloxy groups (preferably substituted or unsubstituted phosphinyloxy groups having 2 to 30 carbon atoms, e.g., diphenoxyphosphinyloxy and dioctyloxyphosphinyloxy); phosphinylamino groups (preferably substituted or unsubstituted phosphinylamino groups having 2 to 30 carbon atoms, e.g., dimethoxyphosphinylamino and dimethylaminophosphinylamino); and silyl groups (preferably substituted or unsubstituted silyl groups having 3 to 30 carbon atoms, e.g., trimethylsilyl, t-butyldimethylsilyl, and phenyldimethylsilyl).

Of the above substituents, those having hydrogen atoms may have their hydrogen atoms replaced by the above substituents. Examples of such substituents include alkylcarbonylaminosulfonyl groups, arylcarbonylaminosulfonyl groups, alkylsulfonylaminocarbonyl groups, arylsulfonylaminocarbonyl groups, methylsulfonylaminocarbonyl groups, p-methylphenylsulfonylaminocarbonyl groups, acetylaminosulfonyl groups, and benzoylaminosulfonyl groups.

The aryl groups represented by R¹, R², and R³ represent substituted or unsubstituted aryl groups. Preferably, the aryl groups have 6 to 50 carbon atoms, more preferably 6 to 30 carbon atoms, particularly preferably 6 to 20 carbon atoms. Preferred examples include phenyl, 2-methylphenyl, 3-methylphenyl, 4-methylphenyl, 2-ethylphenyl, 4-ethylphenyl, 2,4-dimethylphenyl, 2,6-dimethylphenyl, 2,4,6-trimethylphenyl, 1-naphthyl, 2-naphthyl, 2-chlorophenyl, 3-chlorophenyl, 4-chlorophenyl, 2-methoxyphenyl, 3-methoxyphenyl, 4-methoxyphenyl, 2-benzylphenyl, 4-benzylphenyl, 2-methylcarbonylphenyl, and 4-methylcarbonylphenyl.

More preferably, the aryl groups represented by R¹, R², and R³ are phenyl, 2-methylphenyl, 4-methylphenyl, 2-ethylphenyl, 4-ethylphenyl, 2,4-dimethylphenyl, 2,6-dimethylphenyl, 2,4,6-trimethylphenyl, 1-naphthyl, 2-naphthyl, 2-chlorophenyl, 4-chlorophenyl, 2-methoxyphenyl, 3-methoxyphenyl, 4-methoxyphenyl, 2-benzylphenyl, or 4-benzylphenyl, particularly preferably phenyl.

The above aryl groups represented by R¹, R², and R³ may further have a substituent. Examples of substituents include the substituents listed above that the alkyl groups represented by R¹, R², and R³ may have.

The alkoxy groups represented by R¹ and R² represent linear, branched, or cyclic substituted or unsubstituted alkoxy groups. Preferably, the alkoxy groups have 1 to 50 carbon atoms, more preferably 1 to 30 carbon atoms, particularly preferably 1 to 20 carbon atoms. Preferred examples include methoxy, ethoxy, n-propoxy, isopropoxy, cyclopropoxy, butoxy, isobutoxy, t-butoxy, s-butoxy, pentyloxy, isopentyloxy, neopentyloxy, t-pentyloxy, hexyloxy, cyclohexyloxy, heptyloxy, cyclopentyloxy, octyloxy, 2-ethylhexyloxy, nonyloxy, decyloxy, dodecyloxy, tetradecyloxy, hexadecyloxy, octadecyloxy, eicosyloxy, docosyloxy, and triacontyloxy, more preferably methoxy, ethoxy, n-propoxy, isopropoxy, butoxy, isobutoxy, t-butoxy, s-butoxy, pentyloxy, isopentyloxy, neopentyloxy, hexyloxy, cyclohexyloxy, octyloxy, 2-ethylhexyloxy, dodecyloxy, hexadecyloxy, and octadecyloxy, particularly preferably methoxy, ethoxy, n-propoxy, isopropoxy, t-butoxy, pentyloxy, isopentyloxy, hexyloxy, cyclohexyloxy, octyloxy, 2-ethylhexyloxy, dodecyloxy, hexadecyloxy, and octadecyloxy.

The above alkoxy groups represented by R¹ and R² may further have a substituent. Examples of substituents include the substituents listed above that the alkyl groups represented by R¹, R², and R³ may have.

The aryloxy groups represented by R¹ and R² represent substituted or unsubstituted aryloxy groups. Preferably, the aryloxy groups have 6 to 50 carbon atoms, more preferably 6 to 30 carbon atoms, particularly preferably 6 to 20 carbon atoms. Preferred examples include phenoxy, 2-methylphenoxy, 3-methylphenoxy, 4-methylphenoxy, 2-ethylphenoxy, 4-ethylphenoxy, 2,4-dimethylphenoxy, 2,4-di-t-butylphenoxy, 2,6-di-t-butylphenoxy, 2,6-dimethylphenoxy, 2,6-di-t-butyl-4-methylphenoxy, 2,4,6-trimethylphenoxy, 2,4,6-tri-t-butylphenoxy, 1-naphthyloxy, 2-naphthyloxy, 2-chlorophenoxy, 3-chlorophenoxy, 4-chlorophenoxy, 2-methoxyphenoxy, 3-methoxyphenoxy, 4-methoxyphenoxy, 2-benzylphenoxy, 4-benzylphenoxy, 2-methylcarbonylphenoxy, and 4-methylcarbonylphenoxy.

More preferred examples include phenyl, 2,4-di-t-butylphenoxy, and 2,4,6-tri-t-butylphenoxy.

The above aryloxy groups represented by R¹ and R² may further have a substituent. Examples of substituents include the substituents listed above that the alkyl groups represented by R¹, R², and R³ may have.

From the viewpoint of the compatibility between the resin and the phosphorus-containing compound (b1), it is preferred that, in general formula (1), R¹ and R² be alkoxy groups or aryloxy groups, and R³ be an alkyl group or an aryl group.

Examples of divalent or higher-valent groups that serve as a linking group in the compound having a structure represented by general formula (1) include divalent or higher-valent groups derived by removing one or more hydrogen atoms from the substituents listed above for the alkyl groups represented by R¹, R², and R³ (divalent groups if one hydrogen atom is removed from the substituents, or trivalent groups if two hydrogen atoms are removed from the substituents) and combinations thereof. These divalent or higher-valent groups are preferably divalent to hexavalent, more preferably divalent to tetravalent. The above divalent or higher-valent groups are preferably organic groups.

The above divalent or higher-valent groups may further have a substituent. Examples of substituents include the substituents listed above that the alkyl groups represented by R¹, R², and R³ may have.

The above divalent or higher-valent groups preferably have a molecular weight of 10 to 1,000.

Preferred of the above divalent or higher-valent groups and single bonds are single bonds and divalent or higher-valent groups derived by removing one or more hydrogen atoms from amino groups, alkyl groups, aryl groups, bis-aryl groups (arylaryl groups), arylalkylaryl groups, aryloxyaryl groups, alkoxyalkyl groups, alkoxyaryl groups, and alkylaryl groups.

If the compound having a structure represented by general formula (1) is a compound in which a plurality of structures represented by general formula (1) are present in one molecule, it preferably has, in one molecule, 2 to 20 phosphorus atoms, more preferably 2 to 10 phosphorus atoms, even more preferably 2 to 5 phosphorus atoms.

Specific examples of compounds represented by general formula (1) above are given below, although these examples are not intended to limit the present invention.

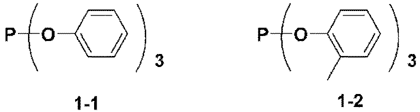

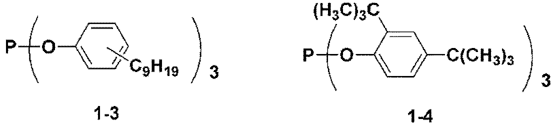

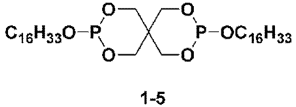

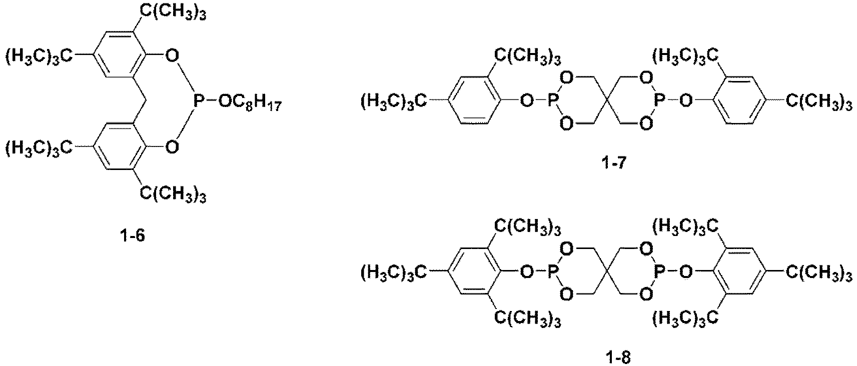

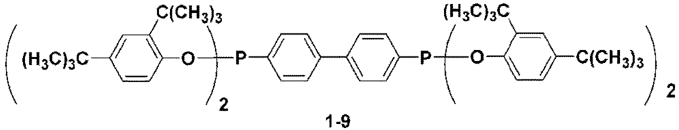

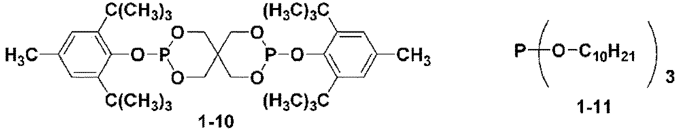

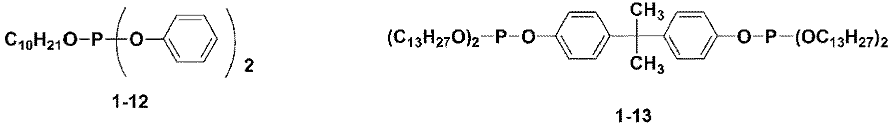

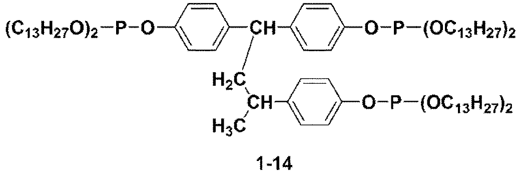

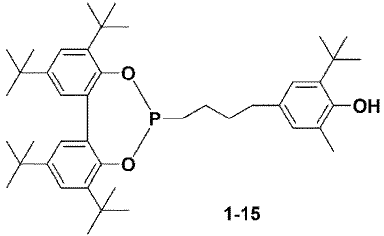

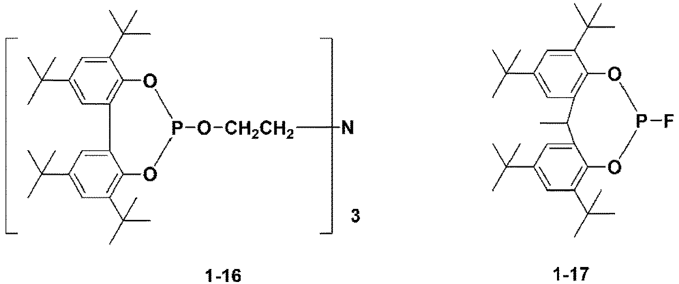

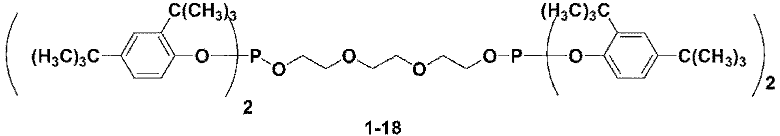

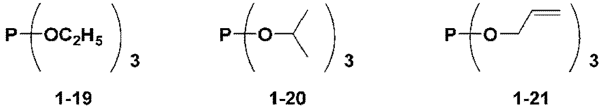

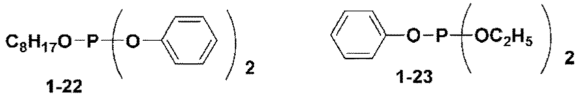

Other compounds suitable for use as the compound having a structure represented by general formula (1) include phosphorous acid ester compounds described in JP2011-527357A.

### Phosphorus-containing compounds (b1) may be used alone or in combination. Thioether Compound (b2)

The thioether compound (b2) is a compound having a structure represented by general formula (2):

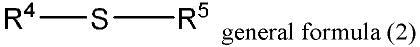

**In** general formula (2), R⁴ and R⁵ represent an alkyl group and may be linked to each other via a divalent or higher-valent group or a single bond.

**In** the present invention, compounds having a structure represented by general formula (2) includes, in addition to compounds represented by general formula (2), compounds (c) and (d) below.
(c) Compounds having a structure in which a monovalent group derived by removing one hydrogen atom from R⁴ or R⁵ is linked to at least one of R⁴ or R⁵ (R⁴ and/or *R⁵*) of one or more (preferably one to three) other compounds represented by general formula (2) via a divalent or higher-valent group or a single bond; and
(d) compounds having a structure in which a divalent or higher-valent group derived by removing a total of two or more hydrogen atoms from at least one group selected from the group consisting of R⁴ and R⁵ (e.g., a divalent group if two hydrogen atoms are removed, or a trivalent group if three hydrogen atoms are removed) is linked to at least one of R¹, R², or R³ of one or more (preferably one to three) other compounds represented by general formula (2) via a divalent or higher-valent group or a single bond.

That is, in the present invention, compounds having a structure represented by general formula (2) are meant to include compounds represented by general formula (2) and compounds having a structure in which a plurality of structures represented by general formula (2) are present in one molecule.

The alkyl groups represented by R⁴ and R⁵ represent linear, branched, or cyclic substituted or unsubstituted alkyl groups. Preferably, the alkyl groups have 1 to 50 carbon atoms, more preferably 2 to 30 carbon atoms, particularly preferably 2 to 20 carbon atoms. Preferred examples include methyl, ethyl, n-propyl, isopropyl, cyclopropyl, butyl, isobutyl, t-butyl, s-butyl, pentyl, isopentyl, neopentyl, t-pentyl, hexyl, cyclohexyl, heptyl, cyclopentyl, octyl, 2-ethylhexyl, nonyl, decyl, dodecyl, tetradecyl, hexadecyl, octadecyl, eicosyl, docosyl, and triacontyl. More preferred are methyl, ethyl, n-propyl, isopropyl, butyl, isobutyl, t-butyl, s-butyl, pentyl, isopentyl, neopentyl, hexyl, cyclohexyl, octyl, 2-ethylhexyl, dodecyl, hexadecyl, and octadecyl. Particularly preferred are methyl, ethyl, n-propyl, isopropyl, butyl, t-butyl, pentyl, isopentyl, hexyl, cyclohexyl, octyl, 2-ethylhexyl, dodecyl, hexadecyl, and octadecyl.

Examples of substituents for the substituted alkyl groups represented by R⁴ and R⁵ include the substituents listed above that the alkyl groups represented by R¹, R², and R³ in general formula (1) may have.

Of the substituted alkyl groups represented by R⁴ and R⁵*,* alkoxycarbonylalkyl groups are preferred. The alkoxycarbonyl groups of the alkoxycarbonylalkyl groups preferably have 2 to 50 carbon atoms, more preferably 5 to 30 carbon atoms, particularly preferably 9 to 20 carbon atoms.

The divalent or higher-valent group serving as a linking group in the compound having a structure represented by general formula (2) is similar to the divalent or higher-valent group described as a linking group in general formula (1) above, and a preferred range is also similar.

Specific examples of compounds represented by general formula (2) above are given below, although these examples are not intended to limit the present invention.

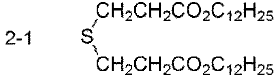

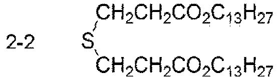

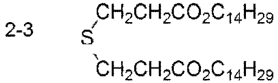

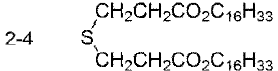

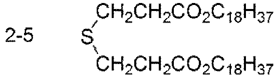

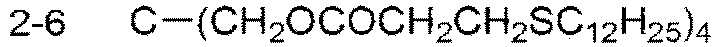

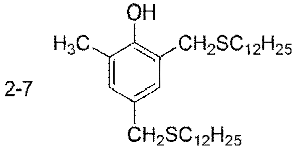

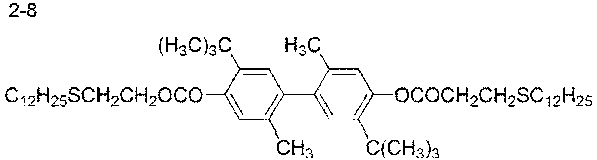

Thioether compounds (b2) may be used alone (not part of the invention) or in combination.

### Hindered Amine Compound (c)

The hindered amine compound (c) is a compound having a structural moiety represented by general formula (3) below.

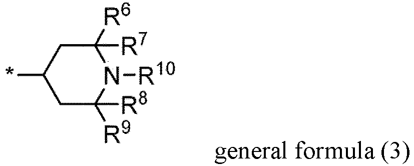

In general formula (3), R⁶ to R⁹ represent a hydrogen atom or an alkyl group having 1 to 12 carbon atoms (preferably 1 to 8 carbon atoms, more preferably 1 to 5 carbon atoms). Specific examples of alkyl groups represented by R⁶ to R⁹ include methyl, ethyl, n-butyl, isopropyl, s-butyl, t-butyl, t-pentyl, t-hexyl, and t-octyl. Preferably, R⁶ to R⁹ are primary (linear) alkyl groups. More preferably, all of R⁶ to R⁹ are primary (linear) alkyl groups (particularly preferably methyl groups).

In general formula (3), R¹⁰ represents a hydrogen atom, an alkyl group having 1 to 18 carbon atoms (preferably 1 to 10 carbon atoms, more preferably 1 to 5 carbon atoms, even more preferably 1 to 3 carbon atoms, further preferably 1 or 2 carbon atoms), or -OR¹¹, where R¹¹ represents a hydrogen atom or a linear, branched, or cyclic alkyl group having 1 to 20 carbon atoms (preferably 1 to 12 carbon atoms). In particular, R¹⁰ is preferably a hydrogen atom, which results in a higher chemical resistance.

In general formula (3), * represents a point of attachment.

The compound having a structural moiety represented by general formula (3) is preferably a compound represented by general formula (3-1) below or a compound having a component (preferably a repeating unit) represented by general formula (3-2) below.

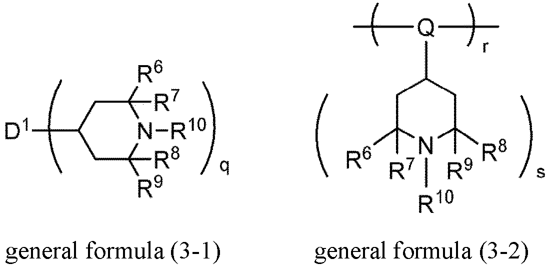

In the formulae, R⁶ to R¹⁰ have the same meanings as R⁶ to R¹⁰, respectively, in general formula (3) above, and preferred ranges are also the same; q represents an integer of 2 or more; D¹ represents a q-valent linking group; s represents 1 or 2; r represents a positive integer, preferably within the range of degrees of polymerization described later; and Q represents an s+2-valent linking group such as a group including an aromatic hydrocarbon group, a group including an imino group (NR^{N}), or a group including a triazine linking group. Specific examples of R^{N} include hydrogen atoms, alkyl groups having 1 to 20 carbon atoms, and piperidyl-containing groups represented by general formula (3).

The linking group represented by D¹ preferably has a molecular weight of 100 to 1,000, more preferably 180 to 600. The linking group represented by Q preferably has a molecular weight of 100 to 1,000, more preferably 180 to 600.

More preferably, the compound having a structural moiety represented by general formula (3) is a compound represented by any of formulae (3-A) to (3-C), (3-E), (3-G), and (3-H) below, a polymer or oligomer having a repeating unit represented by formula (3-D) below (preferably a polymer or oligomer having a repeating unit represented by any of formulae (3D1) to (3D3)), or a polymer or oligomer having a repeating unit represented by formula (3-F) below.

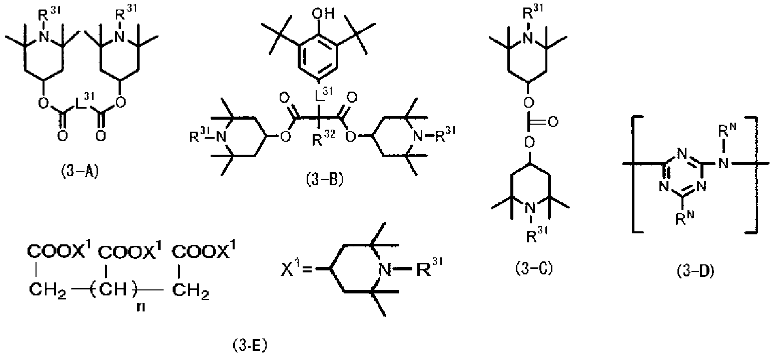

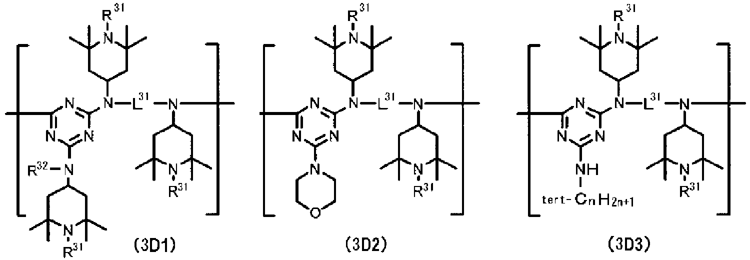

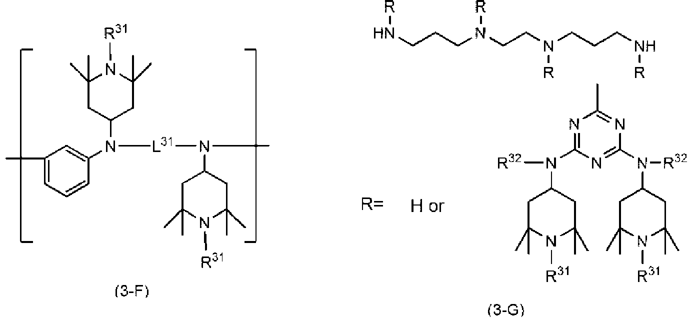

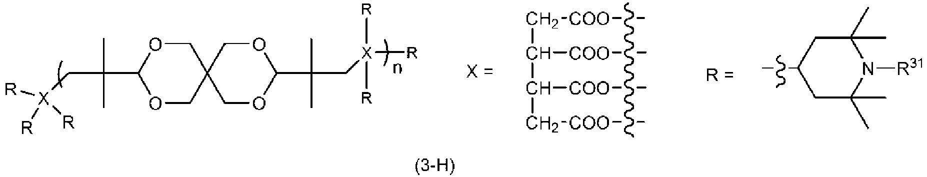

In the formulae, R³¹ has the same meaning as R¹⁰ in general formula (3), and preferred forms are also the same.

R³² represents a hydrogen atom or an alkyl group having 1 to 20 carbon atoms (preferably 1 to 12 carbon atoms, more preferably 1 to 8 carbon atoms, even more preferably 1 to 6 carbon atoms). L³¹ represents a single bond or an alkylene group having 1 to 20 carbon atoms (preferably 1 to 10 carbon atoms). R^{N} has the same meaning as R^{N} in general formula (3-2). n represents an integer of 1 to 20 (preferably 1 to 10) (in formula (3D3), n represents an integer of 4 to 20 (preferably 4 to 10)).

In formula (3-G), at least one R is not H, but a group including triazine. In formula (3-H), the wavy lines represent a point of attachment.

If the compound having a structural moiety represented by general formula (3) is a polymer or oligomer, the number of repeating units (degree of polymerization) is preferably 2 to 100, more preferably 2 to 50, even more preferably 2 to 10. The terminal structures of the polymer or oligomer may each be, for example, but not limited to, a hydrogen atom, a substituted or unsubstituted amino group, or a substituted or unsubstituted triazyl group.

Hindered amine compounds (c) may be used alone (not part of the invention) or in combination.

The amount of the polyester elastomer (a) is preferably 50% by mass or more, more preferably 55% by mass or more, even more preferably 60% by mass or more, further preferably 65% by mass or more, of the resin component forming the layer A (preferably the outermost layer) of the resin layer.

Although the amount of the polyester elastomer (a) may be 100% by mass of the resin component forming the layer A, the amount of the polyester elastomer (a) is preferably 95% by mass or less, more preferably 90% by mass or less, even more preferably 85% by mass or less. If the amount of the polyester elastomer (a) in the layer A falls within the above preferred range, and a soft resin is blended as the remainder, better flexibility can be achieved.

The layer A of the resin layer may have the polyester elastomer (a) alone as the resin component or may further include a component other than the polyester elastomer (a) as the resin component. If the layer A of the resin layer further includes a component other than the polyester elastomer (a) as the resin component, the remainder of the resin component excluding the polyester elastomer (a) preferably includes, as a softer resin, at least one of a polyurethane elastomer (d) or a polyamide elastomer (e).

The polyurethane elastomer (d) may be a common polyurethane elastomer that is applicable to the formation of flexible tubes. The polyamide elastomer (e) may be a common polyamide elastomer that is applicable to the formation of flexible tubes. In the present invention, the polyurethane elastomer (d) preferably has no amide bond, and the polyamide elastomer (e) preferably has no urethane bond.

The inclusion of at least one of the polyurethane elastomer (d) or the polyamide elastomer (e) can improve the adhesiveness between the resin layer including the layer A and the topcoat layer. If the resin component in the layer A of the resin layer includes at least one of the polyurethane elastomer (d) or the polyamide elastomer (e), the total amount of at least one of the polyurethane elastomer (d) or the polyamide elastomer (e) is preferably 5% by mass or more, more preferably 10% by mass or more, even more preferably 20% by mass or more. On the other hand, the total amount of at least one of the polyurethane elastomer (d) or the polyamide elastomer (e) is preferably 50% by mass or less, more preferably 40% by mass or less, even more preferably 35% by mass or less, of the resin component in the layer A of the resin layer. The inclusion of at least one of the polyurethane elastomer (d) or the polyamide elastomer (e) in the above amount can improve the adhesiveness while maintaining desirable elasticity and flexibility and maintaining sufficient chemical resistance.

Polyurethane elastomers (d) may be used alone or in combination. Polyamide elastomers (e) may be used alone or in combination.

In a preferred form of the layer A of the resin layer, at least one of the phosphorus-containing compound (b1) or the thioether compound (b2) (the phosphorus-containing compound (b1) and/or the thioether compound (b2)) is preferably present in a total amount of 0.01 parts by mass or more, more preferably 0.05 parts by mass or more, based on 100 parts by mass of the resin component. On the other hand, the total amount of at least one of the phosphorus-containing compound (b1) or the thioether compound (b2) is preferably 7 parts by mass or less, more preferably 5 parts by mass or less, based on 100 parts by mass of the resin component. If the total amount of at least one of the phosphorus-containing compound (b1) or the thioether compound (b2) falls within the above range, a layer A that provides the desired effect while allowing less of this compound to migrate from the resin can be obtained.

In a preferred form of the layer A of the resin layer, the hindered amine compound (c) is preferably present in an amount of 0.01 parts by mass or more, more preferably 0.1 parts by mass or more, even more preferably 0.5 parts by mass or more, based on 100 parts by mass of the resin component. The upper limit is preferably 7 parts by mass or less, more preferably 5 parts by mass or less. If the amount of the hindered amine compound (c) falls within the above range, a layer A that provides the desired effect while allowing less of this compound to migrate from the resin can be obtained.

In a preferred form of the layer A of the resin layer, the ratio of the total amount of at least one of the phosphorus-containing compound (b1) or the thioether compound (b2) (the amount of the phosphorus-containing compound (b1) and/or the amount of the thioether compound (b2) to the amount of the hindered amine compound (c) is preferably, by mass, 1:50 to 50:1, more preferably 1:30 to 20:1, particularly preferably 1:20 to 10:1. If the ratio of the amounts of these additives falls within the above range by mass, the antagonism between the additives can be inhibited, thus further improving the properties such as chemical resistance.

If the resin layer is composed of a plurality of layers, at least one layer other than the layer A (preferably the outermost layer) preferably contains at least one of the polyester elastomer (a), the polyurethane elastomer (d), or the polyamide elastomer (e) (the polyester elastomer (a), the polyurethane elastomer (d), and/or the polyamide elastomer (e)) (this layer is hereinafter referred to as "layer B"). More preferably, the layer B contains at least the polyurethane elastomer (d), which can improve the adhesiveness between the resin layer including the layer A and the topcoat layer. The layer B preferably contains the polyurethane elastomer (d) as the main component. In this case, the amount of the polyurethane elastomer (d) is preferably 50% by mass or more, more preferably 70% by mass or more, even more preferably 80% by mass or more, further preferably 90% by mass or more, of the resin component in the layer B. It is preferred that all resin component in the layer B be the polyurethane elastomer (d); otherwise, the remainder is preferably at least one of the polyamide elastomer (e) and/or the polyester elastomer (a) (the polyamide elastomer (e) and/or the polyester elastomer (a)).

Alternatively, the layer B may contain the polyamide elastomer (e) as the main component. For example, if the layer B contains the polyamide elastomer (e), the amount of the polyamide elastomer (e) may be 50% by mass or more, or 70% by mass or more, of the resin component. All resin component in the layer B may be the polyamide elastomer (e); otherwise, the remainder is preferably at least one of the polyurethane elastomer (d) or the polyester elastomer (a) (the polyurethane elastomer (d) and/or the polyester elastomer (a)), more preferably the polyurethane elastomer (d).

If the layer B is used as the outermost layer, the layer B preferably includes at least one of the phosphorus-containing compound (b1) or the thioether compound (b2) and the hindered amine compound (c). This can further improve the chemical resistance of the flexible tube. If the layer B is used as an inner layer or interlayer, it may be preferred not to include these compounds, for example, by taking into account adhesiveness to the outer layer, rather than chemical resistance. If the layer B is used as the outermost layer, preferred amounts of the phosphorus-containing compound (b1), the thioether compound (b2), and the hindered amine compound (c), for example, are similar to those of the layer A.

### Resin Composition for Covering Flexible Tube Substrate for Endoscope

The flexible tube according to the present invention is preferably produced using a resin composition for covering a flexible tube substrate for an endoscope. The resin composition for covering a flexible tube substrate for an endoscope includes a polyester elastomer (a), at least one of a phosphorus-containing compound (b1) or a thioether compound (b2), and a hindered amine compound (c) as a mixture thereof,
wherein the phosphorus-containing compound (b1) is a compound having a structure in which only one structure represented by general formula (1) is present in one molecule 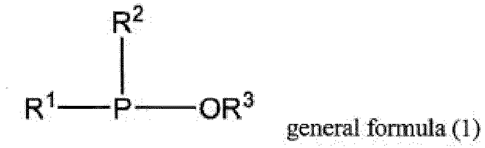 in general formula (1), R¹ and R² represent a substituted or unsubstituted alkyl group, a substituted or unsubstituted aryl group, a substituted or unsubstituted alkoxy group, a substituted or unsubstituted aryloxy group, or a halogen atom; R³ represents a substituted or unsubstituted alkyl group or a substituted or unsubstituted aryl group; and at least two of R¹, R², or R³ may be linked to each other via a divalent or higher-valent group or a single bond.

### Set of Resin Compositions for Covering Flexible Tube Substrate for Endoscope

A flexible tube according to the present invention in which the resin layer is composed of a plurality of layers is preferably produced using a set of resin compositions for covering a flexible tube substrate for an endoscope according to the present invention (a set of resin compositions for covering a flexible tube substrate for an endoscope is hereinafter also simply referred to as "set of resin compositions"). The set of resin compositions according to the present invention includes a resin composition (A) according to the present invention that includes a polyester elastomer (a), at least one of a phosphorus-containing compound (b1) or a thioether compound (b2), and a hindered amine compound (c) and a resin composition (B) that includes at least one of a polyester elastomer (a1), a polyurethane elastomer (d), or a polyamide elastomer (e) (a polyester elastomer (a1), a polyurethane elastomer (d), and/or a polyamide elastomer (e)), wherein the resin composition (A) is for forming an outer layer covering the flexible tube substrate for an endoscope and the resin composition (B) is for forming an inner layer covering the flexible tube substrate for an endoscope. The resin compositions (A) and (B) are separately included in the set of resin compositions.

In the set of resin compositions according to the present invention, the polyester elastomer (a) and the polyester elastomer (a1) may be the same or different.

The resin composition (A) is used to form the layer A of the resin layer of the flexible tube according to the present invention. The resin composition (B), on the other hand, is used to form a layer other than the layer A of the resin layer. The resin composition (B) may include any component present in the resin composition (A) as long as the advantages of the present invention are not impaired.

Preferred ratios of the amount of the polyester elastomer (a), the amount of at least one of the phosphorus-containing compound (b1) or the thioether compound (b2), and the amount of the hindered amine compound (c) in the resin composition (A), for example, are the same as those of the layer A.

The flexible tube according to the present invention preferably includes a resin layer having a two-layer structure composed of one inner layer and one outer layer. In this case, the inner layer is composed of the layer B of the resin layer, whereas the outer layer is composed of the layer A of the resin layer. Preferred formulations for the resin layer are as follows.
· If the adhesiveness between the inner layer and the outer layer is prioritized

| | | Elastomer | Additive |
|---|---|---|---|
| Inner layer | Layer B | PU (PE, PA) | |
| Outer layer | Layer A | PE (PU, PA) | HA, P, SE |

· If the chemical resistance is prioritized

| | | Elastomer | Additive |
|---|---|---|---|
| Inner layer | Layer B | PU (PE, PA) | HA, P, SE |
| Outer layer | Layer A | PE (PU, PA) | HA, P, SE |

PE: polyester elastomer (a)
PU: polyurethane elastomer (d)
PA: polyamide elastomer (e)
HA: hindered amine compound (c)
P: phosphorus-containing compound (b1)
SE: thioether compound (b2)

The constituents in parentheses are optional.

### Physical Properties

To improve the chemical resistance, the molecular weights of the elastomers applied are preferably, but not limited to, 10,000 to 1,000,000, more preferably 20,000 to 500,000, particularly preferably 30,000 to 300,000.

In the present invention, the molecular weight of an elastomer refers to the weight average molecular weight unless otherwise specified. The weight average molecular weight can be determined by GPC as the molecular weight based on polystyrene. An HLC-8220 GPC apparatus (trade name, available from Tosoh Corporation) is used. The eluants used are chloroform for polyester elastomers, N-methyl-2-pyrrolidone (NMP) for polyurethane elastomers, and m-cresol/chloroform (available from Shonan Wako Pure Chemical Co., Ltd.) for polyamide elastomers. The columns used are G3000HXL and G2000HXL (trade names, available from Tosoh Corporation). The temperature is 23°C. The flow rate is 1 mL/min. RI detection is employed.

It is preferred to suitably set the physical properties of the layer B (preferably the inner layer). For example, the layer B preferably has an A hardness (JIS-K7215) of 40 or more, more preferably 50 or more, particularly preferably 60 or more. The upper limit is preferably 98 or less, more preferably 95 or less, particularly preferably 90 or less.

The layer B preferably has a storage modulus E' of 1 MPa or more, more preferably 2 MPa or more, particularly preferably 3 MPa or more. The upper limit is preferably 150 MPa or less, more preferably 100 MPa or less, particularly preferably 50 MPa or less. The layer B preferably has a loss modulus E" of 0.1 MPa or more, more preferably 0.3 MPa or more, particularly preferably 0.5 MPa or more. The upper limit is preferably 20 MPa or less, more preferably 10 MPa or less, particularly preferably 5 MPa or less. The layer B preferably has a loss tangent of 0.01 or more, more preferably 0.03 or more, particularly preferably 0.05 or more. The upper limit is preferably 1 or less, more preferably 0.5 or less, particularly preferably 0.3 or less.

As used herein, the viscoelasticity parameters are measured at 25°C unless otherwise specified. The measurement procedure follows JIS-K7244-4.

It is preferred to suitably set the physical properties of the layer A of the resin layer. For example, the layer A preferably has a D hardness (JIS-K7215) of 40 or more, more preferably 45 or more, particularly preferably 55 or more. The upper limit is preferably 90 or less, particularly preferably 85 or less.

The layer A of the resin layer preferably has a storage modulus E' of 1 MPa or more, more preferably 5 MPa or more, particularly preferably 10 MPa or more. The upper limit is preferably 1 GPa or less, more preferably 500 MPa or less, particularly preferably 300 MPa or less. The layer A of the resin layer preferably has a loss modulus E" of 0.1 MPa or more, more preferably 0.5 MPa or more, particularly preferably 1 MPa or more. The upper limit is preferably 100 MPa or less, more preferably 90 MPa or less, particularly preferably 80 MPa or less. The layer A of the resin layer preferably has a loss tangent of 0.01 or more, more preferably 0.03 or more, particularly preferably 0.05 or more. The upper limit is preferably 1 or less, more preferably 0.5 or less, particularly preferably 0.3 or less.

The layer B preferably has a modulus at 100% elongation of 0.5 MPa or more, more preferably 1.0 MPa or more, particularly preferably 1.5 MPa or more. The upper limit is preferably 20 MPa or less, more preferably 15 MPa or less, particularly preferably 10 MPa or less.

The layer A of the resin layer preferably has a modulus at 100% elongation of 1.0 MPa or more, more preferably 1.5 MPa or more, particularly preferably 2.0 MPa or more. The upper limit is preferably 80 MPa or less, more preferably 70 MPa or less, particularly preferably 65 MPa or less.

As used herein, the modulus is measured at 25°C unless otherwise specified. The measurement procedure follows JIS-K7311.

The resin layer is preferably soluble in 1,1,1,3,3,3-hexafluoro-2-propanol (specific solvent). "Soluble in the specific solvent" means that the resin layer exhibits a degree of solubility of 5% by mass at 20°C. Thus, the technical significance of "soluble in the specific solvent" is that the resin has no three-dimensional (crosslinked) structure, which is preferred because the use of such a resin layer provides good flexibility for a flexible tube for an endoscopic medical device.

The elastomer forming the resin layer is preferably not substantially crosslinked. Here, "not substantially crosslinked" not only means that the resin is not crosslinked, but also means that the resin has no branched structure within the detection limit of, for example, NMR.

It is preferred that the elastomer forming the resin layer (particularly the second layer or the outer layer) according to this embodiment be not substantially crosslinked because the use of such a resin layer provides good flexibility and bending durability for a flexible tube for an endoscopic medical device.

### Topcoat Layer

The topcoat layer (coat layer) 16 is applied to the flexible tube according to this embodiment. Examples of materials the can be applied to the topcoat layer include, but not limited to, urethane coatings, acrylic coatings, fluorinated coatings, silicone coatings, epoxy coatings, and polyester coatings. To achieve high adhesiveness between the resin layer and the topcoat layer and good chemical resistance, which are advantages of this embodiment, urethane coatings, acrylic coatings, and fluorinated coatings are preferred. The topcoat layer may be formed by common processes. One example process involves dissolving the coating component in a predetermined solvent, optionally adding a curing agent to the solution, and hardening the solution. The hardening treatment may be performed, for example, by heating to 100°C to 200°C.

In this embodiment, the topcoat layer is primarily used to protect and add a gloss to the surface of the flexible tube and to impart smoothness and chemical resistance. Thus, a topcoat layer with high elasticity, high surface smoothness, and good chemical resistance is preferred. The topcoat layer alone preferably has a storage modulus E' of 1 MPa or more, more preferably 5 MPa or more, particularly preferably 10 MPa or more. The upper limit is preferably 1 GPa or less, more preferably 500 MPa or less, particularly preferably 300 MPa or less. If the storage modulus E' is higher than or equal to the lower limit, the topcoat layer can provide a surface protection function. If the storage modulus E' is lower than or equal to the upper limit, the resulting flexible tube can maintain its flexibility.

Although a two-layer molded resin layer composed of a soft resin layer (layer B) as an inner layer and a hard resin layer (layer A) as an outer layer is formed in the foregoing embodiment, the hard resin layer may be disposed as the inner layer, and the soft resin layer may be disposed as the outer layer. Although a resin layer having a two-layer configuration has been described by way of example in the foregoing embodiment, the resin layer, in another embodiment, may have a multilayer configuration including two or more layers. The two layers need not be in contact with each other, but may be separated by another functional layer.

Although an electronic endoscope for observation of an image of the condition of a subject captured using an imaging device has been described by way of example in the foregoing embodiment, the present invention is not limited thereto, but may also be applied to an endoscope for observation of the condition of a subject using an optical image guide.

The flexible tube according to the present invention is not limited to endoscope applications, but can also be applied to a wide variety of endoscopic medical devices. For example, the flexible tube according to the present invention can be applied to an endoscope equipped with a clip or wire at the distal end thereof or to a device equipped with a basket or brush and provides its superior effect. Endoscopic medical devices are meant to include a wide variety of flexible medical and diagnostic devices for introduction and use in a body, including medical devices having an endoscope as a basic structure, as described above, and remotely operated medical devices.

### Examples

The present invention will now be described in more detail with reference to the following examples, although these examples should not be construed as limiting the invention.

### Examples and Comparative Examples

Resin compositions (resin mixtures for outer and inner layers) having the formulations shown in Table 1 below (in parts by mass) were prepared and were melt-kneaded in a twin-screw kneader available from Technovel Corporation (product name: KZW15-30MG) at a barrel set temperature of 220°C and a screw rotational speed of 100 rpm. The ejected molten resin strand was cooled in a water bath and was pelletized with a pelletizer to form pelletized samples.

The resulting pelletized samples were introduced into the continuous molding machine shown in Figs. 3 and 4 to produce flexible tubes for endoscopes. Specifically, flexible tube substrates having a diameter of 5.0 mm and a length of 120 cm were covered with the resin mixtures (compositions) for the inner layer and then with the resin mixtures (compositions) for the outer layer in Table 1 below. The resin layer had a thickness of 0.3 mm. The inner-to-outer-layer ratios at the distal and proximal ends were as shown in Table 2 below. For flexible tubes having no inner layer, the inner-to-outer-layer ratio is shown as inner layer:outer layer = 0:100. The resulting flexible tubes were subjected to the following tests. The results are summarized in Table 2.

### Peracetic Acid Resistance

The resin was removed from each flexible tube and was cut to a size of 1 cm × 10 cm to obtain a test specimen. The test specimen was immersed in a 0.3% aqueous peracetic acid solution at 50°C for 150 hours. After the surface of the test specimen was thoroughly washed with water and was then dried at 23°C and 50% RH (relative humidity) for 24 hours, the test specimen was subjected to a tensile test at elongations of 50%, 100%, and 200% (an elongation of 100% means stretching to twice the original length) with a TENSILON RTF-1210 universal material testing machine (trade name, available from A&D Company, Limited). The test specimen was rated on the following scale, where "B" or higher is satisfactory. The results are summarized in Table 2.

### Rating Scale

A: The test specimen was not broken after a tensile test at an elongation of 200%.
B: The test specimen was not broken after a tensile test at an elongation of 100%, but was broken after a tensile test at an elongation of 200%.
C: The test specimen was not broken after a tensile test at an elongation of 50%, but was broken after a tensile test at an elongation of 100%.
D: The test specimen was broken after a tensile test at an elongation of 50%.

### Foaming after Molding

The outer layers of the manufactured flexible tubes were visually observed and were rated on the following scale, where "B" or higher is satisfactory. The results are summarized in Table 2.

### Rating Scale

A: None of the 100 manufactured flexible tubes exhibited foaming in the outer layer after the molding of the outer layer.
B: One to three of the 100 manufactured flexible tubes exhibited a discontinuity in the outer layer due to foaming after the molding of the outer layer.
C: Four to nine of the 100 manufactured flexible tubes exhibited a discontinuity in the outer layer due to foaming after the molding of the outer layer.
D: Ten or more of the 100 manufactured flexible tubes exhibited a discontinuity in the outer layer due to foaming after the molding of the outer layer.

### Adhesiveness Evaluation

The resulting resin mixtures were used to form sheet-shaped articles. The results are summarized in Table 2.

### Sheet Formation Conditions

Each resin composition for the outer layer was heated to 220°C and was pressed at 10 MPa for 30 seconds using a MINI TEST PRESS (available from Toyo Seiki Seisaku-sho, Ltd.) to form a 0.5 mm thick, 10 cm square sheet.

### Topcoat Layer Formation Conditions

The resulting sheet was coated with a topcoat layer under the following conditions.

The material used for the topcoat layer was Obbligato SS0068 (trade name, available from AGC COAT-TECH Co., Ltd.), serving as a base, with a curing agent (available from AGC COAT-TECH Co., Ltd.). This material was applied to the sheet formed as above with a 100 µm thick doctor blade. The coated sample was dried at room temperature (25°C) and was further dried at 80°C for 10 hours to form a resin sheet with a topcoat layer. The topcoat layer had a thickness of 0.02 mm.

### Peracetic Acid Immersion Test on Resin Sheet with Topcoat Layer

The resulting resin sheet with a topcoat was immersed in a 0.3% aqueous peracetic acid solution at 50°C for 50 hours. After the surface was washed with water and was then dried at 23°C and 50% RH for 24 hours, the following adhesiveness evaluation was performed.

### Adhesiveness Evaluation

A polyester tape (available from 3M Company, model No. 850, length: 5 cm, width: 1.5 cm) was attached to the topcoat layer side of the resulting resin sheet with a topcoat layer that had been subjected to the peracetic acid immersion test. The polyester tape was then removed to determine whether the topcoat peeled from the resin sheet. On the following rating scale, "B" or higher is satisfactory.

### Rating Scale

A: The test was performed ten times, and the topcoat layer did not peel at all.
B: The test was performed ten times, and the topcoat layer peeled one to three out of ten times.
C: The test was performed ten times, and the topcoat layer peeled four to nine out of ten times.
D: The test was performed ten times, and the topcoat layer peeled every time.

**Table 1**

| | | Resin for outer layer | | | | | | | | | | | |
|---|---|---|---|---|---|---|---|---|---|---|---|---|---|
| Resin mixture | | A-1 | A-2 | A-3 | A-4 | A-5 | A-6 | A-7 | A-8 | A-9 | A-10 | A-11 | A-12 |
| Elastomer | PE-1 | 100 | | | | | | 100 | 100 | 80 | | | |
| | PE-2 | | 100 | | | 100 | 100 | | | | 90 | 90 | 90 |
| | PE-3 | | | 100 | | | | | | | | | |
| | PE-4 | | | | 100 | | | | | | | | |
| | PU-1 | | | | | | | | | 20 | | | |
| | PU-2 | | | | | | | | | | 10 | | |
| | PA-1 | | | | | | | | | | | 10 | |
| | PA-2 | | | | | | | | | | | | 10 |
| HA | HA-1 | | | | 1 | | | | | | | | |
| | HA-2 | 1 | | | | | | | 0.5 | 1 | | 1 | 1 |
| | HA-3 | | 1 | | | | | | | | | | |
| | HA-4 | | | | | | | 2 | | | | | |
| | HA-5 | | | 1 | | 1 | 1 | | | | 1 | | |
| P | P-1 | 0.5 | | | 0.25 | | | | | | | | |
| | P-2 | | 0.5 | | | 0.075 | 1 | 0.15 | 0.0375 | 0.5 | | 0.25 | |
| SE | S-1 | | | | | 0.075 | 1 | 0.15 | 0.0375 | | | 0.25 | 0.05 |
| | S-2 | | | 0.5 | 0.25 | | | | | | 0.5 | | 0.05 |
| Amount of HA added | | 1 | 1 | 1 | 1 | 1 | 1 | 2 | 0.5 | 1 | 1 | 1 | 1 |
| Total amount of P and SE added | | 0.5 | 0.5 | 0.5 | 0.5 | 0.15 | 2 | 0.3 | 0.075 | 0.5 | 0.5 | 0.5 | 0.1 |
| Ratio of amounts added (amount of HA added /total amount of P and SE added) | | 2.0 | 2.0 | 2.0 | 2.0 | 6.7 | 0.5 | 6.7 | 6.7 | 2.0 | 2.0 | 2.0 | 10 |

**Table 1-continued**

| | | Resin for outer layer | | | | | | | | | | | Resin for inner layer | |
|---|---|---|---|---|---|---|---|---|---|---|---|---|---|---|
| Resin mixture | | A-13 | A-14 | A-15 | A-16 | A-17 | A-18 | C-1 | C-2 | C-3 | C-4 | C-5 | B-1 | B-2 |
| Elastomer | PE-1 | 100 | 100 | 100 | 80 | | | 100 | 100 | 100 | | | | |
| | PE-2 | | | | | | 80 | | | | | | | 10 |
| | PE-3 | | | | | | | | | | | | | |
| | PE-4 | | | | | 100 | | | | | | | | |
| | PU-1 | | | | 20 | | | | | | 100 | 100 | 100 | 90 |
| | PU-2 | | | | | | 20 | | | | | | | |
| | PA-1 | | | | | | | | | | | | | |
| | PA-2 | | | | | | | | | | | | | |
| HA | HA-1 | | | | | 0.1 | 0.2 | | | 2 | 1 | | | |
| | HA-2 | 0.1 | 1 | | 1 | | | | 1 | | | | 0.5 | 0.5 |
| | HA-3 | | | 2 | | | | | | | | | | |
| | HA-4 | | | | | | | | | | | | | |
| | HA-5 | | | | | | | | | | | | | |
| P | P-1 | | 0.05 | | | | 2 | | | | 0.5 | | | |
| | P-2 | | | 0.04 | | | | | | | | | 0.125 | 0.125 |
| SE | S-1 | 0.025 | | | | | | | | | | | 0.125 | 0.125 |
| | S-2 | 0.025 | | | 0.05 | 2 | | 0.5 | | | | | | |
| Amount of HA added | | 0.1 | 1 | 2 | 1 | 0.1 | 0.2 | 0 | 1 | 2 | 1 | 0 | 0.5 | 0.5 |
| Total amount of P and SE added | | 0.05 | 0.05 | 0.04 | 0.05 | 2 | 2 | 0.5 | 0 | 0 | 0.5 | 0 | 0.25 | 0.25 |
| Ratio of amounts added (amount of HA added/total amount of P and SE added) | | 2.0 | 20 | 50 | 20 | 0.05 | 0.1 | 0 | | | 2.0 | | 2.0 | 2.0 |

### Description of Terms in Tables

### Elastomers

(1) Polyester elastomers (the values in parentheses are D hardnesses (JIS-K7215))
   PE-1: Hytrel 7247 (D72), trade name, available from DuPont-Toray Co., Ltd. (weight average molecular weight: 81,000, modulus at 100% elongation: 60.7 MPa)
   PE-2: Hytrel 6347 (D63), trade name, available from DuPont-Toray Co., Ltd. (weight average molecular weight: 82,000, modulus at 100% elongation: 50.1 MPa)
   PE-3: Arnitel UM622 (D62), trade name, available from DSM Japan Engineering Plastics K.K. (weight average molecular weight: 116,000, modulus at 100% elongation: 45.0 MPa)
   PE-4: Pelprene E450B (D78), trade name, available from Toyobo Co., Ltd. (weight average molecular weight: 121,000, modulus at 100% elongation: 70.3 MPa)
(2) Polyurethane elastomers (the values in parentheses are A hardnesses (JIS-K7215))
   PU-1: Miractran E585 (A85), trade name, available from Nippon Miractran Co., Ltd. (weight average molecular weight: 99,000, modulus at 100% elongation: 6.4 MPa)
   PU-2: Elastollan ET 1080 (A80), trade name, available from BASF SE (weight average molecular weight: 124,000, modulus at 100% elongation: 4.0 MPa)
(3) Polyamide elastomers (the values in parentheses are A hardnesses (JIS-K7215))
   PA-1: Pebax 2533 (A75), trade name, available from Arkema Inc. (weight average molecular weight: 208,000, modulus at 100% elongation: 4.4 MPa)
   PA-2: Pebax 3533 (A83), trade name, available from Arkema Inc. (weight average molecular weight: 171,000, modulus at 100% elongation: 6.0 MPa)

### Hindered amine compounds (HA)

HA-1: ADK STAB LA-63P (trade name), available from Adeka Corporation 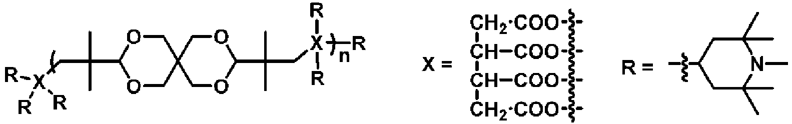 where the wavy lines represent a point of attachment, which also applies to the following formulae; and n represents 1 or 2.

HA-2: Chimassorb 944FDL (trade name), available from BASF SE 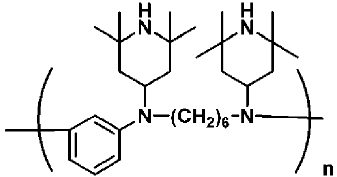 where n represents an integer of 2 to 5.

HA-3: Tinuvin 765 (trade name), available from BASF SE

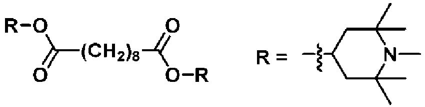

HA-4: Flamestab NOR 116 (trade name), available from BASF SE

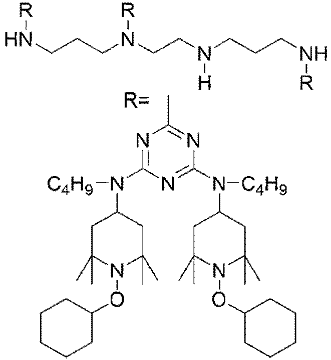

HA-5: Chimassorb 2020FDL (trade name), available from BASF SE 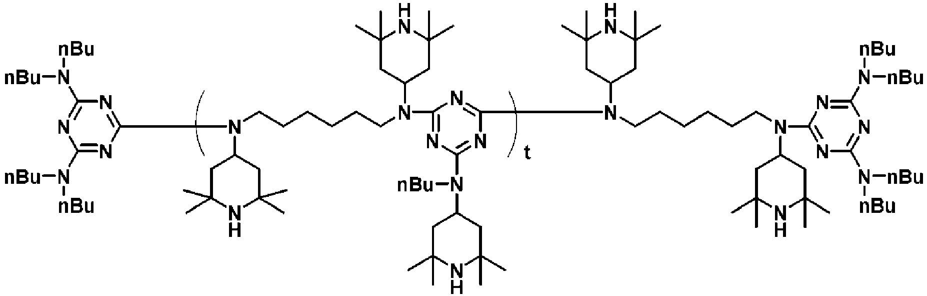 where t represents an integer of 2 to 4, and nBu represents a n-butyl group.

### Phosphorus-containing compounds

P-1: ADK STAB PEP-36 (trade name), available from Adeka Corporation

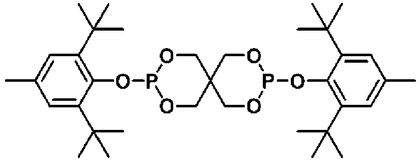

P-2: ADK STAB HP-10 (trade name), available from Adeka Corporation

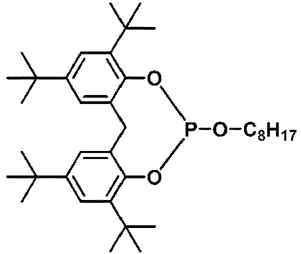

S-1: ADK STAB AO-412S (trade name), available from Adeka Corporation

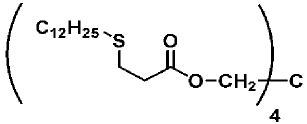

S-2: ADK STAB AO-503 (trade name), available from Adeka Corporation

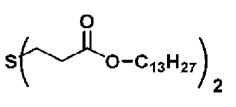

**Table 2**

| | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|
| Outer layer | A-1 | A-2 | A-3 | A-4 | A-5 | A-6 | A-7 | A-8 | A-9 | A-10 | A-11 | A-12 | A-13 |
| Inner layer | None | None | None | None | None | None | None | None | None | None | None | None | None |
| Inner layer:outer layer (proximal end) | 0:100 | 0:100 | 0:100 | 0:100 | 0:100 | 0:100 | 0:100 | 0:100 | 0:100 | 0:100 | 0:100 | 0:100 | 0:100 |
| Inner layer:outer layer (distal end) | 0:100 | 0:100 | 0:100 | 0:100 | 0:100 | 0:100 | 0:100 | 0:100 | 0:100 | 0:100 | 0:100 | 0:100 | 0:100 |
| Peracetic acid resistance | A | A | A | A | A | A | A | A | A | A | A | A | B |
| Foaming after molding | A | A | A | A | B | A | A | B | A | A | A | A | A |
| Adhesiveness Evaluation | B | B | B | B | B | B | B | B | A | A | A | A | B |

| | Example 14 | Example 15 | Example 16 | Example 17 | Example 18 | Example 19 | Example 20 | Comparative Example 1 | Comparative Example 2 | Comparative Example 3 | Comparative Example 4 | Comparative Example 5 | |
|---|---|---|---|---|---|---|---|---|---|---|---|---|---|
| Outer layer | A-1 | A-2 | A-14 | A-15 | A-16 | A-17 | A-18 | C-1 | C-2 | C-3 | C-4 | C-5 | |
| Inner layer | B-1 | B-2 | None | None | None | None | None | None | None | None | None | None | |
| Inner layer:outer layer (proximal end) | 20:80 | 20:80 | 0:100 | 0:100 | 0:100 | 0:100 | 0:100 | 0:100 | 0:100 | 0:100 | 0:100 | 0:100 | |
| Inner layer:outer layer (distal end) | 80:20 | 80:20 | 0:100 | 0:100 | 0:100 | 0:100 | 0:100 | 0:100 | 0:100 | 0:100 | 0:100 | 0:100 | |
| Peracetic acid resistance | A | A | A | A | A | B | B | D | B | B | B | B | |
| Foaming after molding | A | A | B | B | A | B | B | B | D | D | D | D | |
| Adhesiveness Evaluation | A | A | B | B | A | B | A | D | D | D | B | B | |

As can be seen from Table 2, the flexible tubes having resin layers that did not meet the requirements of the present invention were unsatisfactory in terms of at least one of the evaluations items. In contrast, it was found that the flexible tubes according to the present invention contained sufficiently few defects in the resin layer after molding, had the desired sufficient chemical resistance, and achieved a higher adhesiveness between the topcoat layer and the resin layer.

While the present invention has been described in connection with embodiments thereof, we do not intend to limit our invention in any detail of the description unless otherwise specified.

This application claims priority from JP2017-129905 filed in Japan on June 30, 2017.

### Reference Signs List

- 2: electronic endoscope (endoscope)
- 3: insertion section
- 3a: flexible tube
- 3b: angle portion
- 3c: tip portion
- 5: main-body operating section
- 6: universal cord
- 11: spiral tube
- 11a: metal strip
- 12: tubular net
- 13: cap
- 14: flexible tube substrate
- 14a: distal side
- 14b: proximal side
- 15: resin layer
- 16: coat layer
- 17: inner layer
- 18: outer layer
- X: angle portion 3b side (soft)
- Y: main-body operating section 5 side (hard)
- 20: continuous molding machine (manufacturing apparatus)
- 21, 22: extrusion unit
- 21a: screw
- 22a: screw
- 23: head unit
- 24: cooling unit
- 25: transport unit
- 26: control unit
- 28: feed drum
- 29: take-up drum
- 30: joint member
- 31: continuous flexible tube substrate
- 32: nipple
- 33: die
- 34: support
- 35, 36: gate
- 37: molding passage
- 38: resin passage
- 39: soft resin
- 40: hard resin

## Claims

1. A flexible tube for an endoscope, comprising a flexible tube substrate, for an endoscope, that is flexible and tubular and a resin layer covering the flexible tube substrate for an endoscope,
wherein the resin layer includes one or more layers, the layers including a layer A including a polyester elastomer (a) as a resin component, at least one of a phosphorus-containing compound (b1) or a thioether compound (b2), and a hindered amine compound (c),
the phosphorus-containing compound (b1) is a compound having a structure represented by general formula (1),
the thioether compound (b2) is a compound having a structure represented by general formula (2), and
the hindered amine compound (c) is a compound having a structural moiety represented by general formula (3): 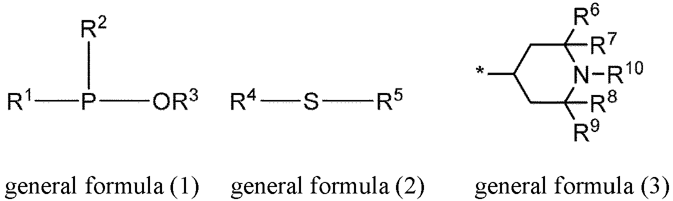 wherein
in general formula (1), R¹ and R² represent a substituted or unsubstituted alkyl group, a substituted or unsubstituted aryl group, a substituted or unsubstituted alkoxy group, a substituted or unsubstituted aryloxy group, or a halogen atom; R³ represents a substituted or unsubstituted alkyl group or a substituted or unsubstituted aryl group; and at least two of R¹, R², or R³ may be linked to each other via a divalent or higher-valent group or a single bond,
in general formula (2), R⁴ and R⁵ represent a substituted or unsubstituted alkyl group and may be linked to each other via a divalent or higher-valent group or a single bond, and
in general formula (3), R⁶ to R⁹ represent a hydrogen atom or an alkyl group having 1 to 12 carbon atoms; R¹⁰ represents a hydrogen atom, an alkyl group having 1 to 18 carbon atoms, or -OR¹¹, wherein R¹¹ represents a hydrogen atom or an alkyl group having 1 to 20 carbon atoms; and * represents a point of attachment;
the recited alkyl and alkoxy groups are linear, branched or cyclic groups.

2. The flexible tube for an endoscope according to claim 1, wherein an amount of the polyester elastomer (a) is 50% by mass or more of the resin component in the layer A.

3. The flexible tube for an endoscope according to claim 1 or 2, wherein the layers include the layer A and a layer B including a polyurethane elastomer (d).

4. The flexible tube for an endoscope according to any one of claims 1 to 3, wherein the layer A further contains, as the resin component, at least one of a polyurethane elastomer (d) or a polyamide elastomer (e).

5. The flexible tube for an endoscope according to any one of claims 1 to 4, wherein a total amount of the phosphorus-containing compound (b1) and the thioether compound (b2) is 0.01 to 5 parts by mass based on 100 parts by mass of the resin component in the layer A.

6. The flexible tube for an endoscope according to any one of claims 1 to 5, wherein an amount of the hindered amine compound (c) is 0.01 to 5 parts by mass, preferably 0.5 to 5 parts by mass based on 100 parts by mass of the resin component in the layer A.

7. The flexible tube for an endoscope according to any one of claims 1 to 6, wherein a ratio of a total amount of the phosphorus-containing compound (b1) and the thioether compound (b2) to an amount of the hindered amine compound (c) in the layer A is 1:50 to 50:1 by mass.

8. The flexible tube for an endoscope according to any one of claims 1 to 7, wherein the hindered amine compound (c) is a compound represented by formula (3-1) or a compound having a component represented by formula (3-2): 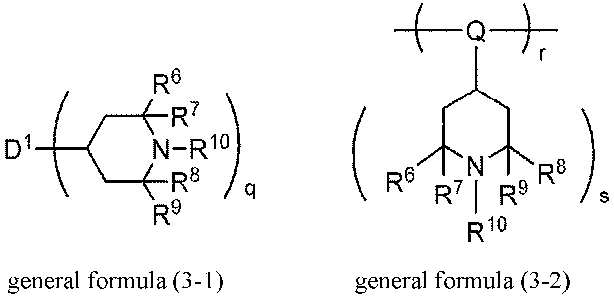 wherein R⁶ to R¹⁰ have the same meanings as R⁶ to R¹⁰, respectively, in general formula (3); q represents an integer of 2 or more; D¹ represents a q-valent linking group; r represents a positive integer; Q represents an s+2-valent linking group; and s represents 1 or 2.

9. The flexible tube for an endoscope according to any one of claims 1 to 8, further comprising a topcoat layer.

10. An endoscopic medical device comprising the flexible tube for an endoscope according to any one of claims 1 to 9.

11. A set of resin compositions for covering a flexible tube substrate for an endoscope, comprising:
a resin composition (A) including a polyester elastomer (a), at least one of a phosphorus-containing compound (b1) or a thioether compound (b2), and a hindered amine compound (c); and
a resin composition (B) including at least one of a polyester elastomer (a1), a polyurethane elastomer (d), or a polyamide elastomer (e),
wherein the resin composition (A) is for forming an outer layer covering the flexible tube substrate for an endoscope and the resin composition (B) is for forming an inner layer covering the flexible tube substrate for an endoscope, and
wherein the phosphorus-containing compound (b1) is a compound having a structure represented by general formula (1), the thioether compound (b2) is a compound having a structure represented by general formula (2), and the hindered amine compound (c) is a compound having a structural moiety represented by general formula (3): 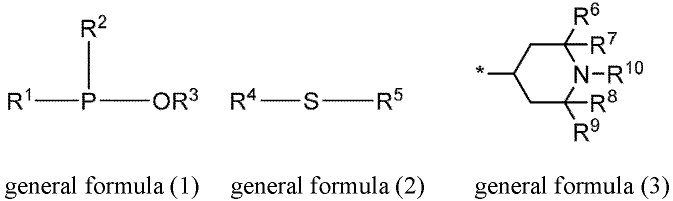 wherein
in general formula (1), R¹ and R² represent an alkyl group, an aryl group, an alkoxy group, an aryloxy group, or a halogen atom; R³ represents an alkyl group or an aryl group; and at least two of R¹, R², or R³ may be linked to each other via a divalent or higher-valent group or a single bond,
in general formula (2), R⁴ and R⁵ represent an alkyl group and may be linked to each other via a divalent or higher-valent group or a single bond, and
in general formula (3), R⁶ to R⁹ represent a hydrogen atom or an alkyl group having 1 to 12 carbon atoms; R¹⁰ represents a hydrogen atom, an alkyl group having 1 to 18 carbon atoms, or -OR¹¹, wherein R¹¹ represents a hydrogen atom or an alkyl group having 1 to 20 carbon atoms; and * represents a point of attachment,
wherein the alkyl group, the aryl group, the alkoxy group and the aryloxy group represented by R¹ and R² in general formula (1) are a substituted or unsubstituted alkyl group, a substituted or unsubstituted aryl group, a substituted or unsubstituted alkoxy group and a substituted or unsubstituted aryloxy group; the alkyl group and the aryl group represented by R³ in general formula (1) are a substituted or unsubstituted alkyl group and a substituted or unsubstituted aryl group; and the alkyl group represented by R⁴ and R⁵ in general formula (2) is a substituted or unsubstituted alkyl group;
the recited alkyl and alkoxy groups are linear, branched or cyclic groups.

12. The set of resin compositions for covering a flexible tube substrate for an endoscope according to claim 11, wherein a ratio of a total amount of the phosphorus-containing compound (b1) and the thioether compound (b2) to an amount of the hindered amine compound (c) is 1:50 to 50:1 by mass.

13. The set of resin compositions for covering a flexible tube substrate for an endoscope according to claim 11, wherein the resin composition (B) includes at least a polyurethane elastomer (d).

## Patentansprüche

1. Flexibles Rohr für ein Endoskop, umfassend ein flexibles Rohrsubstrat für ein Endoskop, das flexibel und rohrförmig ist, und eine Harzschicht, die das flexible Rohrsubstrat für ein Endoskop abdeckt,
wobei die Harzschicht eine oder mehrere Schichten enthält, wobei die Schichten eine Schicht A, die ein Polyesterelastomer (a) als eine Harzkomponente, mindestens eine von einer phosphorhaltigen Verbindung (b1) und einer Thioetherverbindung (b2) und eine gehinderte Aminverbindung (c) enthält, umfassen,
die phosphorhaltige Verbindung (b1) eine Verbindung mit einer Struktur ist, die durch allgemeine Formel (1) dargestellt wird,
die Thioetherverbindung (b2) eine Verbindung mit einer Struktur ist, die durch allgemeine Formel (2) dargestellt wird, und
die gehinderte Aminverbindung (c) eine Verbindung mit einem Strukturabschnitt ist, die durch allgemeine Formel (3) dargestellt wird: 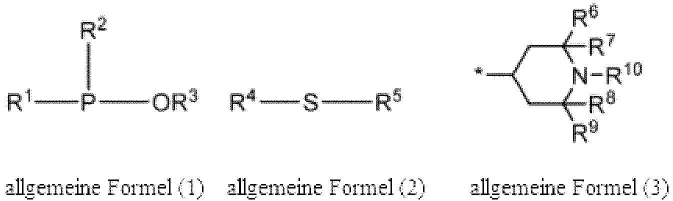
wobei
in allgemeiner Formel (1) R¹ und R² eine substituierte oder unsubstituierte Alkylgruppe, eine substituierte oder unsubstituierte Arylgruppe, eine substituierte oder unsubstituierte Alkoxygruppe, eine substituierte oder unsubstituierte Aryloxygruppe oder ein Halogenatom darstellen; R³ eine substituierte oder unsubstituierte Alkylgruppe oder eine substituierte oder unsubstituierte Arylgruppe darstellt; und mindestens zwei von R¹, R² und R³ via eine divalente oder höherwertige Gruppe oder eine Einfachbindung miteinander verbunden sein können,
in allgemeiner Formel (2) R⁴ und R⁵ eine substituierte oder unsubstituierte Alkylgruppe darstellen und via eine divalente oder höherwertige Gruppe oder eine Einfachbindung miteinander verbunden sein können, und
in allgemeiner Formel (3) R⁶ bis R⁹ ein Wasserstoffatom oder eine Alkylgruppe mit 1 bis 12 Kohlenstoffatomen darstellen; R¹⁰ ein Wasserstoffatom, eine Alkylgruppe mit 1 bis 18 Kohlenstoffatomen oder -OR¹¹ darstellt, wobei R¹¹ ein Wasserstoffatom oder eine Alkylgruppe mit 1 bis 20 Kohlenstoffatomen darstellt; und * einen Befestigungspunkt darstellt;
die genannten Alkyl- und Alkoxygruppen lineare, verzweigte oder zyklische Gruppen sind.

2. Flexibles Rohr für ein Endoskop nach Anspruch 1, wobei eine Menge des Polyesterelastomers (a) 50 Massen-% oder mehr der Harzkomponente in der Schicht A beträgt.

3. Flexibles Rohr für ein Endoskop nach Anspruch 1 oder 2, wobei die Schichten die Schicht A und eine Schicht B, die ein Polyurethanelastomer (d) enthält, umfassen.

4. Flexibles Rohr für ein Endoskop nach einem der Ansprüche 1 bis 3, wobei die Schicht A ferner als die Harzkomponente mindestens eines von einem Polyurethanelastomer (d) und einem Polyamidelastomer (e) enthält.

5. Flexibles Rohr für ein Endoskop nach einem der Ansprüche 1 bis 4, wobei eine Gesamtmenge der phosphorhaltigen Verbindung (b1) und der Thioetherverbindung (b2) 0,01 bis 5 Massenteile auf der Grundlage von 100 Massenteilen der Harzkomponente in der Schicht A beträgt.

6. Flexibles Rohr für ein Endoskop nach einem der Ansprüche 1 bis 5, wobei eine Menge der gehinderten Aminverbindung (c) 0,01 bis 5 Massenteile, bevorzugt 0,5 bis 5 Massenteile auf der Grundlage von 100 Massenteilen der Harzkomponente in der Schicht A beträgt.

7. Flexibles Rohr für ein Endoskop nach einem der Ansprüche 1 bis 6, wobei ein Verhältnis einer Gesamtmenge der phosphorhaltigen Verbindung (b1) und der Thioetherverbindung (b2) zu einer Menge der gehinderten Aminverbindung (c) in der Schicht A 1:50 bis 50:1 nach Masse beträgt.

8. Flexibles Rohr für ein Endoskop nach einem der Ansprüche 1 bis 7, wobei die gehinderte Aminverbindung (c) eine Verbindung, die durch Formel (3-1) dargestellt wird, oder eine Verbindung mit einer Komponente, die durch Formel (3-2) dargestellt wird, ist: 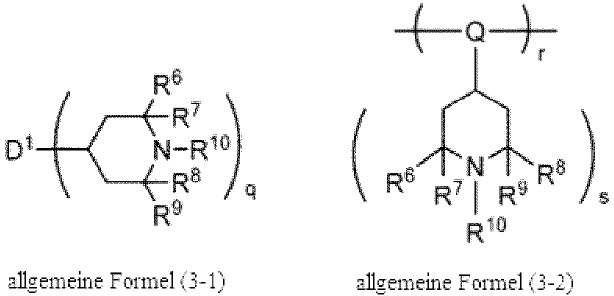 wobei R⁶ bis R¹⁰ die gleichen Bedeutungen wie R⁶ bis R¹⁰ jeweils in der allgemeinen Formel (3) aufweisen; q eine ganze Zahl von 2 oder mehr darstellt; D¹ eine q-wertige Verknüpfungsgruppe darstellt; r eine positive ganze Zahl darstellt; Q eine s+2-wertige Verknüpfungsgruppe darstellt; und s 1 oder 2 darstellt.

9. Flexibles Rohr für ein Endoskop nach einem der Ansprüche 1 bis 8, ferner umfassend eine Deckschicht.

10. Endoskopische medizinische Vorrichtung, umfassend das flexible Rohr für ein Endoskop nach einem der Ansprüche 1 bis 9.

11. Satz von Harzzusammensetzungen zum Abdecken eines flexiblen Röhrensubstrats für ein Endoskop, umfassend:
eine Harzzusammensetzung (A), die ein Polyesterelastomer (a), mindestens eine von einer phosphorhaltigen Verbindung (b1) und einer Thioetherverbindung (b2) und eine gehinderte Aminverbindung (c) enthält; und
eine Harzzusammensetzung (B), die mindestens eines von einem Polyesterelastomer (a1), einem Polyurethanelastomer (d) und einem Polyamidelastomer (e) enthält, wobei die Harzzusammensetzung (A) zum Bilden einer Außenschicht, die das flexible Rohrsubstrat für ein Endoskop abdeckt, dient und die Harzzusammensetzung (B) zum Bilden einer Innenschicht, die das flexible Rohrsubstrat für ein Endoskop abdeckt, dient, und
wobei die phosphorhaltige Verbindung (b1) eine Verbindung mit einer durch die allgemeine Formel (1) dargestellten Struktur ist, die Thioetherverbindung (b2) eine Verbindung mit einer durch die allgemeine Formel (2) dargestellten Struktur ist, und die gehinderte Aminverbindung (c) eine Verbindung mit einem durch die allgemeine Formel (3) dargestellten Strukturabschnitt ist: 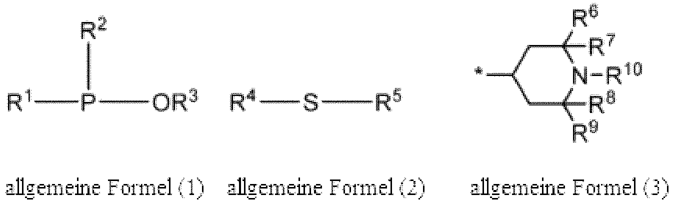
wobei
in allgemeiner Formel (1) R¹ und R² eine Alkylgruppe, eine Arylgruppe, eine Alkoxygruppe, eine Aryloxygruppe oder ein Halogenatom darstellen; R³ eine Alkylgruppe oder eine Arylgruppe darstellt; und mindestens zwei von R¹, R² und R³ via eine divalente oder höherwertige Gruppe oder eine Einfachbindung miteinander verbunden sein können,
in allgemeiner Formel (2) R⁴ und R⁵ eine Alkylgruppe darstellen und via eine divalente oder höherwertige Gruppe oder eine Einfachbindung miteinander verbunden sein können, und
in allgemeiner Formel (3) R⁶ bis R⁹ ein Wasserstoffatom oder eine Alkylgruppe mit 1 bis 12 Kohlenstoffatomen darstellen; R¹⁰ ein Wasserstoffatom, eine Alkylgruppe mit 1 bis 18 Kohlenstoffatomen oder -OR¹¹ darstellt, wobei R¹¹ ein Wasserstoffatom oder eine Alkylgruppe mit 1 bis 20 Kohlenstoffatomen darstellt; und * einen Befestigungspunkt darstellt,
wobei die Alkylgruppe, die Arylgruppe, die Alkoxygruppe und die Aryloxygruppe, die durch R¹ und R² in allgemeiner Formel (1) dargestellt werden, eine substituierte oder unsubstituierte Alkylgruppe, eine substituierte oder unsubstituierte Arylgruppe, eine substituierte oder unsubstituierte Alkoxygruppe und eine substituierte oder unsubstituierte Aryloxygruppe sind; die Alkylgruppe und die Arylgruppe, die durch R³ in allgemeiner Formel (1) dargestellt werden, eine substituierte oder unsubstituierte Alkylgruppe und eine substituierte oder unsubstituierte Arylgruppe sind; und die Alkylgruppe, die durch R⁴ und R⁵ in allgemeiner Formel (2) dargestellt wird, eine substituierte oder unsubstituierte Alkylgruppe ist;
die genannten Alkyl- und Alkoxygruppen lineare, verzweigte oder zyklische Gruppen sind.

12. Satz von Harzzusammensetzungen zum Abdecken eines flexiblen Rohrsubstrats für ein Endoskop nach Anspruch 11, wobei ein Verhältnis einer Gesamtmenge der phosphorhaltigen Verbindung (b1) und der Thioetherverbindung (b2) zu einer Menge der gehinderten Aminverbindung (c) 1:50 bis 50:1 nach Masse beträgt.

13. Satz von Harzzusammensetzungen zum Abdecken eines flexiblen Röhrensubstrats für ein Endoskop nach Anspruch 11, wobei die Harzzusammensetzung (B) mindestens ein Polyurethanelastomer (d) enthält.

## Revendications

1. Tube flexible pour un endoscope, comprenant un substrat de tube flexible, pour un endoscope, qui est flexible et tubulaire, et une couche de résine recouvrant le substrat de tube flexible pour un endoscope,
dans lequel la couche de résine inclut une ou plusieurs couches, les couches incluant une couche A incluant un élastomère de polyester (a) en tant que composant de résine, au moins un d'un composé contenant de phosphore (b1) ou d'un composé de thioéther (b2), et un composé d'amine entravée (c),
le composé contenant de phosphore (b1) est un composé ayant une structure représentée par formule générale (1),
le composé de thioéther (b2) est un composé ayant une structure représentée par formule générale (2), et
le composé d'amine entravée (c) est un composé ayant une partie structurelle représentée par formule générale (3) : 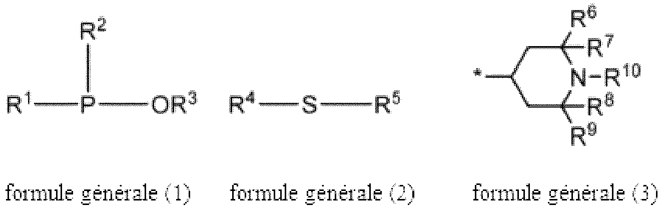
dans lequel
dans formule générale (1), R¹ et R² représentent un groupe alkyle substitué ou non substitué, un groupe aryle substitué ou non substitué, un groupe alkoxy substitué ou non substitué, un groupe aryloxy substitué ou non substitué, ou un atome d'halogène ; R³ représente un groupe alkyle substitué ou non substitué ou un groupe aryle substitué ou non substitué ; et au moins deux de R¹, R² ou R³ peuvent être liés entre eux par un groupe divalent ou de valence supérieure ou par une liaison simple,
dans formule générale (2), R⁴ et R⁵ représentent un groupe alkyle substitué ou non substitué et peuvent être liés l'un à l'autre par un groupe divalent ou de valence supérieure ou par une liaison simple, et
dans formule générale (3), R⁶ à R⁹ représentent un atome d'hydrogène ou un groupe alkyle ayant de 1 à 12 atomes de carbone ; R¹⁰ représente un atome d'hydrogène, un groupe alkyle ayant de 1 à 18 atomes de carbone, ou -OR¹¹, dans laquelle R¹¹ représente un atome d'hydrogène ou un groupe alkyle ayant de 1 à 20 atomes de carbone ; et * représente un point de liaison ;
les groupes alkyle et alkoxy cités sont des groupes linéaires, ramifiés ou cycliques.

2. Tube flexible pour un endoscope selon la revendication 1, dans lequel une quantité de l'élastomère de polyester (a) est de 50 % en masse ou plus du composant de résine dans la couche A.

3. Tube flexible pour un endoscope selon la revendication 1 ou la revendication 2, dans lequel les couches incluent la couche A et une couche B incluant un élastomère de polyuréthane (d).

4. Tube flexible pour un endoscope selon l'une quelconque des revendications 1 à 3, dans lequel la couche A contient en outre, en tant que composant de résine, au moins un d'un élastomère de polyuréthane (d) ou d'un élastomère de polyamide (e).

5. Tube flexible pour un endoscope selon l'une quelconque des revendications 1 à 4, dans lequel une quantité totale du composé contenant de phosphore (b1) et du composé de thioéther (b2) est de 0,01 à 5 parties en masse sur la base de 100 parties en masse du composant de résine dans la couche A.

6. Tube flexible pour un endoscope selon l'une quelconque des revendications 1 à 5, dans lequel une quantité du composé d'amine entravée (c) est de 0,01 à 5 parties en masse, de préférence de 0,5 à 5 parties en masse sur la base de 100 parties en masse du composant de résine dans la couche A.

7. Tube flexible pour un endoscope selon l'une quelconque des revendications 1 à 6, dans lequel un rapport entre une quantité totale du composé contenant de phosphore (b1) et du composé de thioéther (b2) et une quantité du composé d'amine entravée (c) dans la couche A est de 1:50 à 50:1 en masse.

8. Tube flexible pour un endoscope selon l'une quelconque des revendications 1 à 7, dans lequel le composé d'amine entravée (c) est un composé représenté par formule (3-1) ou un composé ayant un composant représenté par formule (3-2) : 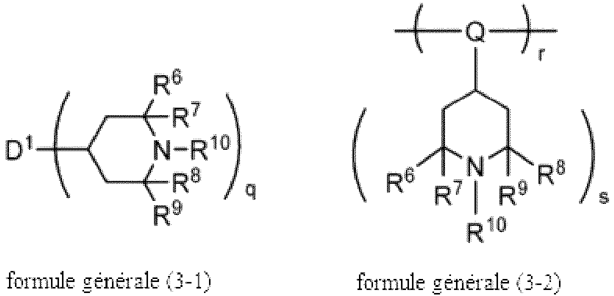 dans lequel R⁶ à R¹⁰ ont les mêmes significations que R⁶ à R¹⁰, respectivement, dans formule générale (3) ; q représente un entier de 2 ou plus ; D¹ représente un groupe de liaison q-valent ; r représente un entier positif ; Q représente un groupe de liaison s+2-valent ; et s représente 1 ou 2.

9. Tube flexible pour un endoscope selon l'une quelconque des revendications 1 à 8, comprenant en outre une couche de finition.

10. Dispositif médical endoscopique comprenant le tube flexible pour un endoscope selon l'une quelconque des revendications 1 à 9.

11. Ensemble de compositions de résine pour recouvrir un substrat de tube flexible pour un endoscope, comprenant :
une composition de résine (A) incluant un élastomère de polyester (a), au moins un d'un composé contenant de phosphore (b1) ou d'un composé de thioéther (b2), et un composé d'amine entravée (c) ; et
une composition de résine (B) incluant au moins un d'un élastomère de polyester (a1), d'un élastomère de polyuréthane (d) ou d'un élastomère de polyamide (e),
dans lequel la composition de résine (A) est destinée à former une couche externe recouvrant le substrat de tube flexible pour un endoscope et la composition de résine (B) est destinée à former une couche interne recouvrant le substrat de tube flexible pour un endoscope, et
dans lequel le composé contenant de phosphore (b1) est un composé ayant une structure représentée par formule générale (1), le composé de thioéther (b2) est un composé ayant une structure représentée par formule générale (2), et le composé d'amine entravée (c) est un composé ayant une partie structurelle représentée par formule générale (3) : 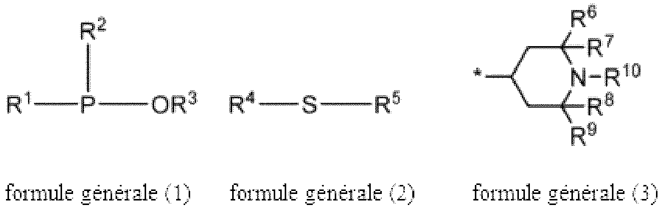
dans lequel
dans formule générale (1), R¹ et R² représentent un groupe alkyle, un groupe aryle, un groupe alkoxy, un groupe aryloxy ou un atome d'halogène ; R³ représente un groupe alkyle ou un groupe aryle ; et au moins deux de R¹, R² ou R³ peuvent être liés entre eux par un groupe divalent ou de valence supérieure ou par une liaison simple,
dans formule générale (2), R⁴ et R⁵ représentent un groupe alkyle et peuvent être liés l'un à l'autre par un groupe divalent ou de valence supérieure ou par une liaison simple, et
dans formule générale (3), R⁶ à R⁹ représentent un atome d'hydrogène ou un groupe alkyle ayant de 1 à 12 atomes de carbone ; R¹⁰ représente un atome d'hydrogène, un groupe alkyle ayant de 1 à 18 atomes de carbone, ou -OR¹¹, dans laquelle R¹¹ représente un atome d'hydrogène ou un groupe alkyle ayant de 1 à 20 atomes de carbone ; et * représente un point de liaison,
dans lequel le groupe alkyle, le groupe aryle, le groupe alkoxy et le groupe aryloxy représentés par R¹ et R² dans formule générale (1) sont un groupe alkyle substitué ou non substitué, un groupe aryle substitué ou non substitué, un groupe alkoxy substitué ou non substitué et un groupe aryloxy substitué ou non substitué ; le groupe alkyle et le groupe aryle représentés par R³ dans formule générale (1) sont un groupe alkyle substitué ou non substitué et un groupe aryle substitué ou non substitué ; et le groupe alkyle représenté par R⁴ et R⁵ dans formule générale (2) est un groupe alkyle substitué ou non substitué ;
les groupes alkyle et alkoxy cités sont des groupes linéaires, ramifiés ou cycliques.

12. Ensemble de compositions de résine pour recouvrir un substrat de tube flexible pour un endoscope selon la revendication 11, dans lequel un rapport entre une quantité totale du composé contenant de phosphore (b1) et du composé de thioéther (b2) et une quantité du composé d'amine entravée (c) est de 1:50 à 50:1 en masse.

13. Ensemble de compositions de résine pour recouvrir un substrat de tube flexible pour un endoscope selon la revendication 11, dans lequel la composition de résine (B) inclut au moins un élastomère de polyuréthane (d).
